# EUROPEAN PATENT APPLICATION

(11) **EP 4 483 925 A1**
(43) Date of publication of application: **01.01.2025**
(21) Application number: 23759946.9
(22) Date of filing: 21.02.2023
(51) Int. Cl.: A61M 5/178, A61M 5/28, A61M 5/31, C12M 3/00, A61J 3/00

(54) **SYRINGE FOR CRYOPRESERVATION**

(30) Priority: 25.02.2022 JP 2022028246
(71) Applicant: Otsuka Pharmaceutical Factory, Inc., Naruto-shi, Tokushima 772-8601 (JP)
(72) Inventor: HASHIMOTO, Kazumasa, Naruto-shi, Tokushima 772-8601 (JP); ONODERA, Kyoka, Naruto-shi, Tokushima 772-8601 (JP)
(74) Representative: D Young & Co LLP
(86) International application number: PCT/JP2023/006108
(87) International publication number: WO 2023/162945

(57) **Abstract**

According to the present invention, a syringe comprising a barrel having a spout and an opening; a gasket slidably accommodated in the barrel; a plunger connected to the gasket; and a cap that seals the spout of the barrel, wherein the opening of the barrel is sealed only with the gasket. The syringe is excellent in usability, durability, and safety.

## Description

### Technical Field

The present invention relates to a syringe for cryopreservation, a prefilled syringe, and a method for cryopreserving a subject substance.

### Background Art

In recent years, opportunities for using various cells are increasing in the medical field, the field of pharmaceutical manufacturing technology, and the field of testing and research of medicine, pharmacy, agriculture, and biology or the like. In particular, due to the development of stem cell research including iPS cells (induced pluripotent stem cells), there are increasing expectations for practical application of cell transplantation therapy in the field of regenerative medicine.

Therapeutic cells such as stem cells used in regenerative medicine are often administered to patients through culture processes such as selection, concentration, and proliferation depending on the purpose. Therefore, therapeutic cells often have opportunities to be transported between medical facilities and cell processing and culturing facilities or preserved at those facilities, before being administered to patients. In order to maintain the viability of the therapeutic cells and maintain the therapeutic ability of the cells during transportation and preservation, therapeutic cells generally need to be frozen at a very low temperature such as -80°C or less. Accordingly, containers storing therapeutic cells are also required to withstand extremely low temperatures during transportation and preservation of therapeutic cells.

Further, therapeutic cells will eventually be injected into patients, and syringes are generally used for injecting the therapeutic cells to patients. Replacing the therapeutic cells from containers for transportation and preservation into syringes is laborious, and there are risks of the mix-up of therapeutic cells in the process, microbial contamination, and foreign matter contamination. Therefore, in the field of regenerative medicine, there is a need for prefilled syringes in which therapeutic cells are stored in advance, in view of the work efficiency and the sanitary conditions. When the contents of prefilled syringes are cells, freezing at very low temperatures is required during transportation and preservation, as described above, and therefore the syringes of course need to withstand such very low temperatures.

Patent Document 1 discloses a syringe for freezing and thawing therapeutic cell mixtures, comprising a barrel made of a cyclic olefin polymer or the like for reasons such as transparency. Further, Patent Document 2 discloses a syringe for storing and dispensing cryopreserved cells, which is made of a cycloolefin polymer or cycloolefin copolymer as a biocompatible material. However, it is disclosed that the syringe of Patent Document 1 requires a special structure such as a cap or a seal near the opening of a barrel, since the gasket alone is not sufficient to protect the sterility in the barrel during preservation at a temperature lower than -80°C. Further, the syringe of Patent Document 2 also requires a cap at the opening of a barrel as an essential configuration although it includes a gasket in the barrel. This is probably because, similarly to the syringe of Patent Document 1, it is impossible to prevent contamination into the barrel with microorganisms or foreign matter without the cap at the opening of the barrel in addition to the gasket in the barrel under very low temperature conditions during cell freezing.

The 17th edition of the Japanese Pharmacopoeia states that it is necessary to confirm that plastic pharmaceutical containers used for injections are "tight containers free from microorganisms". Here, the term "tight containers" refer to "containers that are impervious to solid or liquid foreign matter and can prevent the loss, efflorescence, deliquescence, or evaporation of the pharmaceuticals stored under normal handling, transportation, or preservation". Accordingly, it is essential for plastic syringes, especially for medical use, to have at least the ability to prevent the intrusion of microorganisms, solid foreign matter, or liquid foreign matter (that is, integrity).

Known cell syringes for cryopreservation require a special structure for maintaining the integrity during freezing and thawing such as a cap and a seal at the opening of the barrel, which is not found in general syringes, as mentioned above. However, since such a special structure needs to be removed during use of syringes, and the user must also perform the operation to connect the gasket and the plunger, it takes time and effort to use syringes, thereby increasing the work load. Thus, there is a risk of hindering prompt medical practice especially in the medical field. In addition, during these operations, a strong force may be partially applied to the barrel, the gasket, or the plunger of syringes, which may cause distortion or cracking, leading to contamination of syringes with microorganisms or foreign matter. Further, there is also a problem that the need for a special structure such as a cap or a seal, which is not found in general syringes, leads to an increase in manufacturing costs of syringes.

Meanwhile, it is generally conceivable to increase the diameter of the gasket relative to the inner diameter of the barrel, in order to impart the integrity to the structure of syringes. However, increasing the diameter of the gasket relative to the inner diameter of the barrel causes a reduction in slidability of the gasket within the barrel, which may lead to a deterioration in usability of syringes.

As another method for improving the integrity of a syringe, there is a technique of forming the gasket from a rubber-based material that has excellent adhesion to the inner wall of the barrel, or laminating the gasket with a rubber-based material. However, rubber-based materials generally vitrify and become brittle at very low temperatures, and there is a concern that the durability may decrease. As still another method, there is also a technique of coating the inside of the barrel or the gasket with a silicone compound. Since the use of a silicone compound may also contribute to the improvement of slidability, they are often used for coating syringes. However, when the contents of syringes contain proteins, the silicone compound may cause aggregation of proteins, which may lead to serious safety problems especially in syringes intended for medical use.

### Prior Art Documents

### Patent Documents

[Patent Document 1] Japanese unexamined Patent Application Publication (Translation of PCT Application) No. 2020-510477
[Patent Document 2] Japanese unexamined Patent Application Publication (Translation of PCT Application) No. 2008-507563

### Summary of the Invention

### Object to be Solved by the Invention

It is an object of the present invention is to provide a syringe for cryopreservation that can maintain its integrity during preservation not only at room temperature but also at a very low temperature and heating and is excellent in usability, durability, and safety, without requiring a special structure such as a cap or a seal at the opening of the barrel or using a silicone compound or a rubber-based material.

### Means to Solve the Object

As a result of dedicated studies in order to solve the aforementioned problems, the inventor has found that a syringe that can be used for cryopreservation that is excellent in usability, durability, and safety can be provided even by forming a syringe with a barrel having a spout and an opening, a gasket slidably accommodated in the barrel, a plunger connected to the gasket, and a cap that seals the spout of the barrel, and sealing the opening of the barrel only with the gasket, thereby accomplishing the present invention.

That is, the present invention is as specified by the following items.
(1) A syringe for cryopreservation, comprising:
   a barrel having a spout and an opening;
   a gasket slidably accommodated in the barrel;
   a plunger connected to the gasket; and
   a cap that seals the spout of the barrel,
   wherein the opening of the barrel is sealed only with the gasket.
(2) The syringe according to (1) above, wherein the plunger is connected to the gasket while having a gap from the barrel.
(3) The syringe according to (1) or (2) above, wherein the cap comprises a substrate and a mantle tube fitted from a circumferential edge of the substrate to an outer circumferential surface of the spout of the barrel.
(4) The syringe according to (3) above, wherein the cap further comprises a projection protruding from an inner center of the substrate and fitted into an inner circumferential surface of the spout of the barrel.
(5) The syringe according to (4) above, wherein the mantle tube of the cap protrudes towards the barrel side more than the projection of the cap.
(6) The syringe according to any one of (1) to (5) above, wherein a maximum load when the gasket is slid in the barrel at a speed of 100 mm/minute is 8 N or less.
(7) The syringe according to any one of (1) to (6) above, wherein a ratio (B/A) of a diameter (B) of the gasket to an inner diameter (A) of the barrel is 1 ≤ (B/A) < 1.050.
(8) The syringe according to any one of (1) to (7) above, wherein the gasket comprises two or more gasket lips.
(9) The syringe according to any one of (1) to (8) above, wherein a ratio (X/Y) of a shrinkage rate (X) at which a resin forming the barrel is cooled from a room temperature to a certain temperature to a shrinkage rate (Y) at which a resin forming the gasket is cooled from the room temperature to the certain temperature is 1 ≤ (X/Y) < 1.3.
(10) The syringe according to any one of (1) to (9) above, wherein the barrel is formed from a material having an absorbed energy in a tensile impact test under liquid nitrogen cooling conditions of 0.23 J or more.
(11) The syringe according to any one of (1) to (10) above, wherein the barrel and the gasket are each independently formed from a fluororesin or a polyethylene resin.
(12) The syringe according to any one of (1) to (11) above, wherein the gasket and the plunger are integrally molded.
(13) The syringe according to any one of (1) to (12) above, wherein the syringe further comprises a finger grip.
(14) The syringe according to (13) above, wherein the finger grip is detachable.
(15) The syringe according to (13) or (14) above, wherein the outer circumferential surface of the finger grip and the outer circumferential surface of the barrel are partially substantially flush with each other.
(16) The syringe according to any one of (1) to (15) above, wherein the syringe is further stored in an outer bag.
(17) The syringe according to any one of (1) to (16) above, wherein the syringe is for storing a cell, a nucleic acid, or a protein.
(18) The syringe according to (17) above, wherein the cell is a mammalian cell.
(19) The syringe according to any one of (1) to (18) above, wherein the syringe is for use in regenerative medicine.
(20) The syringe according to any one of (1) to (19) above, wherein the syringe is used at -80°C or less for cryopreservation.
(21) The syringe according to any one of (1) to (19) above, wherein the syringe is used at -150°C or less for cryopreservation.
(22) A prefilled syringe for cryopreservation, wherein the prefilled syringe stores a content in the syringe according to any one of (1) to (21) above.
(23) The prefilled syringe according to (22) above, wherein the prefilled syringe is used at -80°C or less for cryopreservation.
(23') The prefilled syringe according to (22) above, wherein the subject substance is cryopreserved in the syringe at - 80°C or less.
(24) The prefilled syringe according to (22) above, wherein the prefilled syringe is used at -150°C or less for cryopreservation.
(24') The prefilled syringe according to (22) above, wherein the subject substance is cryopreserved in the syringe at - 150°C or less.
(25) A method for cryopreserving a subject substance, comprising cryopreserving the subject substance in the syringe according to any one of (1) to (21) above.
(26) The method according to (25) above, wherein the cryopreservation is a cryopreservation at -80°C or less.
(27) The method according to (25) above, wherein the cryopreservation is a cryopreservation at -150°C or less.

Further, for example, the following can be exemplified. (28) The syringe according to any one of (1) to (21) above, the prefilled syringe according to any one of (22) to (24) above, or the method according to any one of (25) to (27) above, wherein a ratio (C/B) of a thickness (C) of the gasket to a diameter (B) of the gasket is 0.10 or less. (29) The syringe according to any one of (1) to (21) and (28) above, the prefilled syringe according to any one of (22) to (24) above, or the method according to any one of (25) to (27) above, wherein the thickness (C) of the gasket is 0.7 mm or less.

As another embodiment of the present invention, the following can be mentioned, for example.
(30) A method for cryopreserving a subject substance, comprising the steps of:
   providing a subject substance to be cryopreserved,
   providing a syringe comprising: a barrel having a spout and an opening; a gasket slidably accommodated in the barrel; a plunger connected to the gasket; and a cap that seals the spout of the barrel, wherein the opening of the barrel is sealed only with the gasket;
   storing the subject substance in the barrel of the syringe and sealing the barrel with the gasket and the cap; and
   putting the syringe storing the subject substance under a desired low temperature to cryopreserve the subject substance.
(31) A method for administering a subject substance, comprising the steps of:
   providing the subject substance to be administered;
   providing a syringe comprising: a barrel having a spout and an opening; a gasket slidably accommodated in the barrel; a plunger connected to the gasket; and a cap that seals the spout of the barrel, wherein the opening of the barrel is sealed only with the gasket;
   storing the subject substance in the barrel of the syringe and sealing the barrel with the gasket and the cap;
   cryopreserving the subject substance by putting the syringe storing the subject substance under a desired low temperature;
   thawing the subject substance by putting the syringe under a desired high temperature; and
   administering the thawed subject substance to a subject animal in need of administration of the subject substance from the syringe.
(32) The method according to (31) above, wherein the subject animal is a mammal (for example, a human).
(33) The method according to (31) or (32) above, wherein the desired high temperature is a temperature of 10°C or more, room temperature, or 37°C.
(34) The method according to any one of (30) to (33) above, wherein the desired low temperature is a temperature of - 20°C or less.
(35) The method according to any one of (30) to (33) above, wherein the desired low temperature is a temperature of - 80°C or less.
(36) The method according to any one of (30) to (33) above, wherein the desired low temperature is a temperature of - 150°C or less.
(37) Use of the syringe according to any one of (1) to (21), (28) and (29) above, for cryopreserving a subject substance.
(38) The use according to (37) above, wherein the cryopreservation is a cryopreservation at -80°C or less.
(39) The use according to (37) above, wherein the cryopreservation is a cryopreservation at -150°C or less.
(40) A kit comprising the syringe according to any one of (1) to (21), (28) and (29) above, and an instruction for use describing the method according to any one of (25) to (27) and (30) to (36) above (that is, for performing the method according to any one of (25) to (27) and (30) to (36) above).

The syringe of the present invention is a syringe which use is limited as "for use in the cryopreservation" of a subject substance. Therefore, it can be said that the present invention is a use limited invention.

### Effect of the Invention

According to the present invention, a syringe for cryopreservation that has an integrity during preservation not only at room temperature but also at a very low temperature and is excellent in usability, durability, and safety can be provided without using a special structure for enhancing the integrity of the syringe, a silicone compound and a rubber-based material.

### Brief Description of Drawings

[Figure 1] Figure 1 is a schematic view showing a configuration of the syringe of this embodiment during preservation.
[Figure 2] Figure 2 is a schematic view showing a configuration of the cap of this embodiment.

### Mode of Carrying Out the Invention

Hereinafter, an embodiment for carrying out the present invention (which will be hereinafter referred to simply as "this embodiment") will be described in detail. The following embodiment is shown as an example for describing the present invention, and the present invention is not limited to the embodiment. The present invention can be appropriately modified and implemented within the scope of the gist thereof.

The configuration of the syringe of this embodiment will be described. The following configuration is an aspect of the syringe of this embodiment, and the syringe of the present invention is not limited by the following configuration.

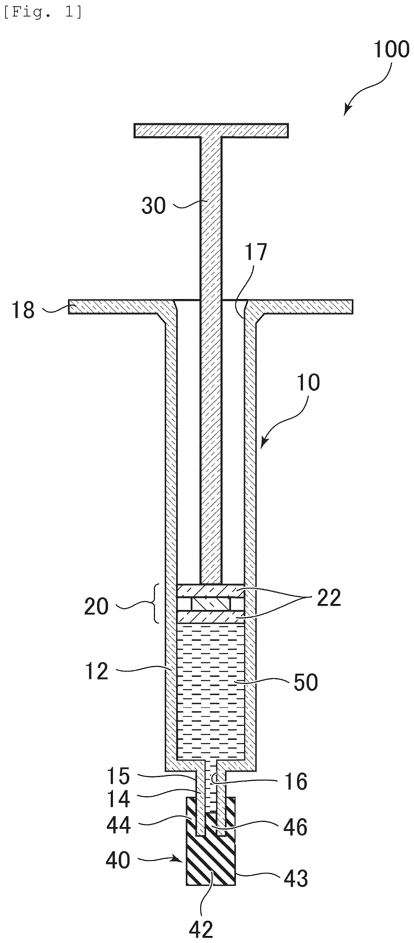

Figure 1 is a schematic view showing a configuration of the syringe of this embodiment during preservation. As shown in Figure 1, the syringe 100 comprises a barrel 10, a gasket 20, a plunger 30, and a cap 40. The syringe 100 is a so-called prefilled syringe that stores contents 50 in a enclosed state. The syringe 100 is stored in an outer bag 60 (not shown). In the present invention, the term "enclosed state" means that the exchange of liquids and solids inside and outside the barrel is blocked.

As shown in Figure 1, the barrel 10 has a cylindrical body 12, and the contents 50 are stored therein. Further, the cylindrical body 12 has a spout 14 for spouting (discharging) the contents 50 on its tip and has an opening 17 into which the gasket 20 is inserted on the other end. Further, the opening 17 has a finger grip 18.

As shown in Figure 1, the cap 40 is attached on the spout 14 of the cylindrical body 12 during preservation of the syringe 100. The cap 40 is detached when the syringe 100 is used, and an injection needle (not shown) is attached on the spout 14. The spout 14 consists of a portion having a smaller diameter then that of the cylindrical body 12.

The gasket 20 is connected to one end of the plunger 30 and slidably installed within the barrel 10. The gasket 20 is accommodated in the barrel 10 through the opening 17 of the barrel 10, adheres closely the inner surface of the barrel 10, and is pushed to move in a direction from the opening 17 toward the spout 14 when the syringe 100 is used (that is, the gasket 20 slides within the barrel 10). When the gasket 20 is thus pushed to move, the contents 50 in the barrel 10 are subjected to a pressure in the same direction. As described above, an injection needle (not shown) is attached on the spout 14 when the syringe 100 is used, and the contents 50 subjected to the pressure by the gasket 20 are spouted through the injection needle (not shown) via the spout 14. The gasket 20 is composed of two gasket lips 22.

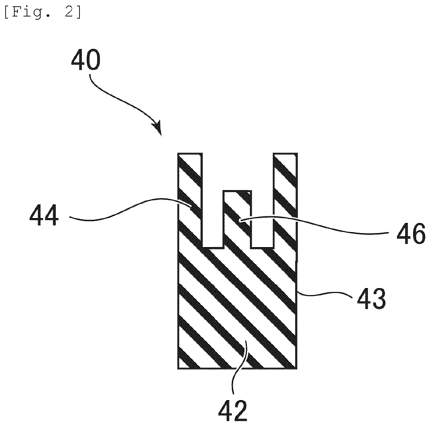

Figure 2 is a schematic view showing a configuration of the cap of this embodiment. The cap 40 comprises a substrate 42, a mantle tube 44 fitted from a circumferential edge 43 of the substrate 42 to an outer circumferential surface 15 of the spout 14 of the barrel 10, and a projection 46 protruding from an inner center of the substrate 42 and fitted into an inner circumferential surface 16 of the spout 14 of the barrel 10. The mantle tube 44 is fitted and adheres closely to the outer circumferential surface 15 of the spout 14 of the barrel 10, and the projection 46 is fitted and adheres closely into the inner circumferential surface 16 of the spout 14 of the barrel 10, thereby sealing the spout 14 of the barrel 10.

Hereinafter, each member will be described.

In the syringe of the present invention, the opening of the barrel is sealed only by the gasket. The term "seal" means to block the exchange of liquids and solids inside and outside the barrel to prevent the intrusion of microorganisms, solid foreign matter, or liquid foreign matter. The syringe of the present invention can maintain the integrity of the syringe by sealing the opening of the barrel only with the gasket at room temperature and even a very low temperature. Therefore, the syringe of the present invention can be composed free from a special member for sealing the opening of the barrel such as a cap or a seal other than the gasket in the barrel. That is, the syringe of the present invention can be provided as a syringe with high usability that does not require the work to remove such a special cap or seal or the work to connect the gasket to the plunger in use. Further, the syringe of the present invention can be configured as a syringe being free or substantially free of (not using or substantially not using) a silicone compound for securing good slidability or good integrity. That is, the syringe of the present invention can be provided as a syringe with high safety in which the contents are not adversely affected by a silicone compound or contaminated by foreign matter. Further, the syringe of the present invention can be configured as a syringe being free or substantially free of (not using or substantially not using) rubber-based materials for securing good slidability or good integrity, e.g., elastomers such as thermoplastic elastomers, natural rubber, or synthetic rubber. That is, the syringe of the present invention can be provided as a syringe with high durability that does not become brittle even at a very low temperature. Here, the silicone compound is not particularly limited, as long as it is a compound having a siloxane bond, and may be in any state of a liquid such as silicone oil, a solid such as silicone resin, or a semi-solid mixture thereof. Further, the natural rubber or synthetic rubber is not particularly limited, and examples thereof include isoprene rubber, polyisoprene/styrene butadiene rubber, butadiene rubber, butyl rubber, and silicone rubber. Further, "substantially free of" or "substantially not using" means that it is allowed to comprise or use if it is an embodiment that does not negatively affect the contents of the syringe (for example, embodiment that does not contact the contents of the syringe), or a minute amount that does not negatively affect (for example, a minute amount in a level that does not cause aggregation, degeneration, or inactivation of the contents). The syringe of the present invention is a syringe in which at least the exchange of liquids and solids inside and outside the barrel is blocked to prevent the intrusion of microorganisms, solid foreign matter, or liquid foreign matter into the barrel but can be an aspect in which the exchange of gases is also completely or almost completely blocked.

In the syringe of the present invention, the gasket is slidably accommodated in the barrel configured to store a subject substance, but the position of the gasket in the barrel is not particularly limited and can be appropriately adjusted depending on the presence or absence or the amount of the contents (subject substances) stored in the barrel. For example, when nothing is stored in the barrel, the gasket may be accommodated adhering closely to the spout side of the barrel, when the amount of the contents in the barrel is small, the gasket may be accommodated on the spout side of the barrel, or when the amount of the contents in the barrel is large, the gasket may be accommodated on the opening side of the barrel.

In the syringe of the present invention, the spout of the barrel is sealed by the cap. The term "seal" means to block the exchange of liquids and solids inside and outside the barrel to prevent the intrusion of microorganisms, solid foreign matter, or liquid foreign matter. The shape of the cap is not limited, as long as the cap adheres closely to the spout of the barrel to secure the integrity of the syringe. In an aspect, the cap comprises a substrate and a mantle tube fitted from a circumferential edge of the substrate to an outer circumferential surface of the spout of the barrel. In an aspect, the cap comprises a substrate, a mantle tube fitted from a circumferential edge of the substrate to an outer circumferential surface of the spout of the barrel, and a projection protruding from an inner center of the substrate and fitted into an inner circumferential surface of the spout. In an aspect, the mantle tube of the cap protrudes towards the barrel side more than the projection of the cap. In an aspect, the substrate is disc-shaped. In an aspect, the mantle tube is in a hollow cylindrical shape. In an aspect, the projection is in a cylindrical shape. In an aspect, the diameter of the outer circumferential surface of the spout of the barrel and the diameter of the inner circumferential surface of the mantle tube of the cap are substantially the same. In an aspect, the diameter of the inner circumferential surface of the spout of the barrel and the diameter of the outer circumferential surface of the projection of the cap are substantially the same. The term "substantially the same" means to allow the state of having a margin of error that does not hinder the adhesion of the cap to the barrel spout in addition to the state of being the same.

In an aspect, the substrate, the mantle tube, and the projection are integrally formed from the same material. In an aspect, the cap and the barrel are formed from the same material.

In the syringe of the present invention, examples of the ratio (B/A) of a diameter (B) of the gasket to an inner diameter (A) of the barrel include 1 ≤ (B/A), 1 < (B/A), 1.002 ≤ (B/A), 1.005 ≤ (B/A), 1.006 ≤ (B/A), 1.010 ≤ (B/A), 1.015 ≤ (B/A), and 1.020 ≤ (B/A), such as (B/A) < 1.100, (B/A) < 1.090, (B/A) < 1.080, (B/A) < 1.070, (B/A) < 1.060, (B/A) < 1.050, (B/A) < 1.040, (B/A) < 1.030, and (B/A) < 1.025, that is, 1 ≤ (B/A) < 1.100, 1 ≤ (B/A) < 1.090, 1 ≤ (B/A) < 1.080, 1 ≤ (B/A) < 1.070, 1 ≤ (B/A) < 1.060, 1 ≤ (B/A) < 1.050, 1 ≤ (B/A) < 1.040, 1 ≤ (B/A) < 1.030, and 1 ≤ (B/A) < 1.025, 1.002 ≤ (B/A) < 1.050, 1.002 ≤ (B/A) < 1.030, 1.005 ≤ (B/A) < 1.050, 1.005 ≤ (B/A) < 1.030, 1.006 ≤ (B/A) < 1.050, 1.006 ≤ (B/A) < 1.030, preferably 1 ≤ (B/A) < 1.050, and more preferably 1 ≤ (B/A) < 1.030. When the ratio (B/A) falls within such a range, the syringe can maintain its integrity at room temperature and very low temperatures, and a syringe having good slidability of the gasket inside the barrel can be produced. When the ratio (B/A) is excessively large, the resistance of the gasket slid in the barrel increases, and the slidability deteriorates. Meanwhile, when the ratio (B/A) is excessively small, a gap occurs between the barrel and the gasket, and the opening of the barrel cannot be sealed only by the gasket. In particular, the integrity of the syringe cannot be maintained theoretically unless 1 ≤ (B/A).

Here, the slidability of the gasket slid in the barrel can be evaluated by a known method. For example, it can be evaluated by measuring the maximum load (N) at a moving distance of 40 mm at a moving speed of 100 mm/minute using a tensile tester. The slidability that can be evaluated in this way is preferably a maximum load of 10 N or less, more preferably 8 N or less, further preferably 7 N or less, particularly preferably 6 N or less, in which case, the slidability can be determined to be good. That is, a syringe with the maximum load (N) when the gasket is slid in the barrel falling within such a range can be provided as a syringe having good slidability and thus excellent usability.

In the present invention, the gasket may be composed of one gasket lip or may be composed of two or more gasket lips. Providing two or more gasket lips can further enhance the integrity of the syringe. The thickness of the gasket in the portion in contact with the inner surface of the barrel (size in the sliding contact direction of the sliding contact portion of the gasket and the inner surface of the barrel) is not particularly limited but there is a concern that as the contact area of the gasket with the inner surface of the barrel increases as the thickness of the gasket increases, the friction increases, and the slidability deteriorates. Examples of the thickness (C) of the gasket include 0.1 mm or more, 0.2 mm or more, 0.3 mm or more, 0.4 mm or more, 0.5 mm or more, 0.6 mm or more, 0.7 mm or more, 0.8 mm or more, and 0.9 mm or more and 1.3 mm or less, 1.2 mm or less, 1.1 mm or less, 1.0 mm or less, less than 1.0 mm, 0.9 mm or less, 0.8 mm or less, 0.7 mm or less, 0.6 mm or less, 0.5 mm or less, and 0.4 mm or less, that is, 0.1 mm to 0.9 mm, 0.1 mm to 0.7 mm, 0.1 mm to 0.6 mm, 0.1 mm to 0.5 mm, 0.2 mm to 0.8 mm, 0.2 mm to 0.7 mm, 0.2 mm to 0.6 mm, 0.2 mm to 0.5 mm, 0.3 mm to 1.2 mm, 0.4 mm to 1.1 mm, 0.5 mm to 1.0 mm, 0.3 mm to 0.7 mm, 0.2 mm to 0.6 mm, 0.3 mm to 0.5 mm, and 0.4 mm to 0.6 mm, and it is preferably 0.7 mm or less, and more preferably 0.5 mm or less.

Further, examples of the ratio (C/B) of a thickness (C) of the gasket to a diameter (B) of the gasket include 0.01 or more, 0.02 or more, 0.03 or more, 0.04 or more, 0.05 or more, 0.06 or more, and 0.07 or more and 0.15 or less, 0.14 or less, 0.13 or less, 0.12 or less, 0.11 or less, 0.10 or less, 0.09 or less, and 0.08 or less, that is, 0.01 ≤ (C/B) ≤ 0.15, 0.01 ≤ (C/B) ≤ 0.10, 0.01 ≤ (C/B) ≤ 0.09, 0.01 ≤ (C/B) ≤ 0.08, 0.02 ≤ (C/B) ≤ 0.14, 0.03 ≤ (C/B) ≤ 0.14, 0.03 ≤ (C/B) ≤ 0.13, 0.02 ≤ (C/B) ≤ 0.12, 0.02 ≤ (C/B) ≤ 0.11, 0.02 ≤ (C/B) ≤ 0.10, 0.02 ≤ (C/B) ≤ 0.09, 0.02 ≤ (C/B) ≤ 0.08, 0.03 ≤ (C/B) ≤ 0.10, 0.03 ≤ (C/B) ≤ 0.09, and 0.03 ≤ (C/B) ≤ 0.08, and it is preferably 0.10 or less, and more preferably 0.08 or less.

When the gasket is composed of one gasket lip, the thickness of the gasket lip is preferably within such a range, and when the gasket is composed of two or more gasket lips, the total thickness of the two or more gasket lips is preferably within such a range.

Further, in the present invention, the thickness of the gasket refers to the thickness of the portion in contact with the inner surface of the barrel of the gasket (that is the sliding contact portion) or of the portion that can be substantially in contact with the inner surface of the barrel (that is the portion that can be substantially in sliding contact). For example, in case the shape of the sliding contact portion is a flat surface or approximate flat surface as shown in Fig. 1, it refers to the thickness of the flat surface or approximate flat surface, and in case the shape of the sliding contact portion is a curved surface with an arc-shape or approximate arc-shape, or the like, it refers to the chord length or less of the arc or approximate arc. Here, the chord length of the arc or approximate arc refers to the distance between the end points of the arc or approximate arc. Further, in the present invention, the shape of sliding contact portion, or portions other than the sliding contact portion is not limited as long as it does not impair the effect of the present invention.

In the syringe of the present invention, examples of the ratio (X/Y) of a shrinkage rate (X) at which a resin forming the barrel is cooled from a room temperature to a certain low temperature to a shrinkage rate (Y) at which a resin forming the gasket is cooled from the room temperature to the low temperature include 1 ≤ (X/Y), 1 ≤ (X/Y) < 1.5, 1 ≤ (X/Y) < 1.45, 1 ≤ (X/Y) < 1.4, 1 ≤ (X/Y) < 1.35, 1 ≤ (X/Y) < 1.3, 1 ≤ (X/Y) < 1.25, 1 ≤ (X/Y) < 1.2, 1 ≤ (X/Y) < 1.15, 1 ≤ (X/Y) < 1.1, and 1 ≤ (X/Y) < 1.05, most preferably (X/Y) = 1. In this way, when the shrinkage rate (X) at which a resin forming the barrel is cooled from a room temperature to a certain low temperature and the shrinkage rate (Y) at which a resin forming the gasket is cooled from the room temperature to the low temperature are the same or in a close range, the integrity of the syringe can be maintained during preservation not only at room temperature but also at very low temperatures. When (Y) is larger than (X), a gap occurs between the barrel and the gasket when cooled to the low temperature, and thus the integrity of the syringe cannot be maintained theoretically, unless 1 ≤ (X/Y). Further, when (X) is excessively large with respect to (Y), there is a fear that the gasket or barrel may be broken because they cannot withstand the shrinkage pressure of the barrel when cooled to the low temperature, or the gasket may be disengaged from the barrel.

Further, the same applies to not only the relationship between the barrel and the gasket but also to the relationship between the barrel and the cap. That is, examples of the ratio (Z/X) of a shrinkage rate (Z) at which a resin forming the cap is cooled from a room temperature to a low temperature to a shrinkage rate (X) at which a resin forming the barrel is cooled from the room temperature to the certain low temperature in the syringe of the present invention include 1 ≤ (Z/X), 1 ≤ (Z/X) < 1.5, 1 ≤ (Z/X) < 1.45, 1 ≤ (Z/X) < 1.4, 1 ≤ (Z/X) < 1.35, 1 ≤ (Z/X) < 1.3, 1 ≤ (Z/X) < 1.25, 1 ≤ (Z/X) < 1.2, 1 ≤ (Z/X) < 1.15, 1 ≤ (Z/X) < 1.1, and 1 ≤ (Z/X) < 1.05, most preferably (Z/X) = 1. In this way, when the shrinkage rate (X) at which a resin forming the barrel is cooled from a room temperature to a certain low temperature and the shrinkage rate (Z) at which a resin forming the cap is cooled from the room temperature to the low temperature are the same or in a close range, the integrity of the syringe can be maintained during preservation not only at room temperature but also at very low temperatures. When (X) is larger than (Z), a gap occurs between the barrel and the cap when cooled to the low temperature, and thus the integrity of the syringe cannot be maintained theoretically, unless 1 ≤ (Z/X). Further, when (Z) is excessively large with respect to (X), there is a fear that the cap or barrel may be broken because they cannot withstand the shrinkage pressure of the barrel when cooled to the low temperature, or the cap may be disengaged from the barrel.

In the syringe of the present invention, at least the material (resin) forming the barrel, for example, has an absorbed energy in a tensile impact test under liquid nitrogen cooling conditions of 0.23 J or more, 0.24 J or more, 0.25 J or more, 0.26 J or more, 0.27 J or more, 0.28 J or more, 0.29 J or more, 0.30 J or more, 0.31 J or more, 0.32 J or more, 0.33 J or more, 0.34 J or more, and 0.35 J or more, preferably 0.25 J or more, more preferably 0.30 J or more. Here, a larger absorbed energy (J) means a higher impact resistance. A syringe having a sufficient impact resistance can be provided during preservation not only at room temperature but also at very low temperatures by forming the barrel that is a member particularly susceptible to the impact from the outside among the constituents of the syringe from a material having a high impact resistance at least within such a range. Further, not only the barrel but also the gasket, the plunger, the cap, the finger grip, and/or the outer bag are preferably formed from the same material having a high impact resistance, so that a sufficient impact resistance can be imparted also to each member during preservation not only at room temperature but also at very low temperatures. Examples of the method for conducting the tensile impact test under liquid nitrogen cooling conditions include a method of sufficiently cooling a test piece, crosshead, and fixing jig with a liquid nitrogen (-196°C) and measuring the absorbed energy using a commercially available tensile impact test.

In the syringe of the present invention, the barrel and the gasket can be formed from a resin, particularly, each independently from a fluororesin or a polyethylene resin. Further, the cap, the plunger, the finger grip, the outer bag can be formed from a resin, particularly, each independently from a fluororesin or a polyethylene resin.

Specifically, examples of the fluororesin in the present invention can include polytetrafluoroethylene (PTFE), tetrafluoroethylene (TFE)-ethylene copolymer (ETFE), tetrafluoroethylene (TFE)-hexafluoropropylene (HFP)-based copolymer (FEP), and TFE-perfluoroalkyl vinyl ether (PAVE)-based copolymer (PFA).

The "TFE-HFP-based copolymer" means a copolymer containing at least TFE and HFP. That is, the "TFE-HFP-based copolymer" includes ternary copolymers such as a copolymer of TFE, HFP, and vinyl fluoride (VF) (TFE/HFP/VF copolymer), a copolymer of TFE, HFP, and vinylidene fluoride (VDF) (TFE/HFP/VDF copolymer), and a copolymer of TFE, HFP, and perfluoro(alkyl vinyl ether) (PAVE) (TFE/HFP/PAVE copolymer), quaternary copolymers such as a copolymer of TFE, HFP, VF, and VDF (TFE/HFP/VF/VDF copolymer), a copolymer of TFE, HFP, VF, and PAVE (TFE/HFP/VF/PAVE copolymer), and a copolymer of TFE, HFP, VDF, and PAVE (TFE/HFP/VDF/PAVE copolymer), and quinary copolymers such as a copolymer of TFE, HFP, VF, VDF, and PAVE (TFE/HFP/VF/VDF/PAVE copolymer), in addition to binary copolymers such as a copolymer of TFE and HFP (TFE/HFP copolymer; FEP).

The TFE-HFP-based copolymer is preferably a TFE/HFP copolymer and a TFE/HFP/PAVE copolymer. The mass ratio of TFE and HFP in such a TFE/HFP copolymer is preferably 80 to 97/3 to 20, more preferably 84 to 92/8 to 16. When the mass ratio of HFP in the TFE/HFP copolymer is lower than the aforementioned numerical range, the formability is poor, and when it is higher than the aforementioned numerical range, the mechanical strength and the hardness are low, and the dimensional stability is poor. Further, the mass ratio of TFE, HFP, and PAVE in the TFE/HFP/PAVE copolymer is preferably 70 to 97/3 to 20/0.1 to 10, more preferably 81 to 92/5 to 16/0.3 to 5. When the mass ratio of HFP and PAVE in the TFE/HFP/PAVE copolymer is lower than the aforementioned numerical range, the formability is poor, and when it is higher than the aforementioned numerical range, the mechanical strength and the hardness are low, and the dimensional stability is poor.

The "TFE-PAVE-based copolymer " means a copolymer containing at least TFE and PAVE. That is, the "TFE-PAVE-based copolymer" includes ternary copolymers such as a copolymer of TFE, PAVE, and hexafluoropropylene (HFP) (TFE/PAVE/HFP copolymer), a copolymer of TFE, PAVE, and vinylidene fluoride (VDF) (TFE/PAVE/VDF copolymer), and a copolymer of TFE, PAVE, and chlorotrifluoroethylene (CTFE) (TFE/PAVE/CTFE copolymer), quaternary copolymers such as a copolymer of TFE, PAVE, HFP, and VDF (TFE/PAVE/HFP/VDF copolymer), a copolymer of TFE, PAVE, HFP, and CTFE (TFE/PAVE/HFP/CTFE copolymer), and a copolymer of TFE, PAVE, VDF, and CTFE (TFE/PAVE/VDF/CTFE copolymer), and quinary copolymers such as a copolymer of TFE, PAVE, HFP, VDF, and CTFE (TFE/PAVE/HFP/VDF/CTFE copolymer), in addition to binary copolymers such as a copolymer of TFE and PAVE (TFE/PAVE copolymer; PFA).

The PAVE constituting the PAVE unit is not specifically limited, and examples thereof can include perfluoro(methyl vinyl ether) [PMVE], perfluoro(ethyl vinyl ether) [PEVE], perfluoro(propyl vinyl ether) [PPVE], perfluoro(butyl vinyl ether), perfluoro(pentyl vinyl ether), perfluoro(hexyl vinyl ether), and perfluoro(heptyl vinyl ether).

The mass ratio of TFE and PAVE in the TFE-PAVE-based copolymer is preferably 90 to 98/2 to 10, more preferably 92 to 97/3 to 8. When the mass ratio of PAVE in the TFE-PAVE-based copolymer is lower than the aforementioned numerical range, the formability is poor, and when it is higher than the aforementioned numerical range, the mechanical strength and the hardness are low, and the dimensional stability is poor.

Further, examples of the fluororesin in the present invention may include a fluororesin in which the total number of nonfluorinated group ends (for example, functional groups such as -COOH, -CH₂OH, -CONH₂, and -COOCH₃ associated with -COF, -COOH, and water) and -CF₂H group ends in the fluororesin per 1 × 10⁶ of carbon atoms is 70 or less, 50 or less, 35 or less, 20 or less, or 10 or less, and a fluororesin containing one or more -CF₃ end groups and having a more stabilized end structure. Further, the fluororesin is possible to not contain -CF₂ end group, and in case of not containing -CF₂ end group, the number of non-fluorinated group ends in the fluororesin can be 70 or less, 50 or less, 35 or less, 20 or less or 10 or less per 1 × 10⁶ of carbon atoms. The numbers of -COF, -COOH, -COOH associated with water, -CH₂OH, -CONH₂, -COOCH₃, and -CF₂H per 1 × 10⁶ of carbon atoms can be calculated by FT-IR, and the -CF₃ end groups can be analyzed by high-temperature ¹⁹F NMR measurement. These fluororesins can be used particularly preferably when the contents are cells, proteins, or nucleic acids, since they can suppress the adsorption of the cells, nucleic acids, or proteins stored in the barrel onto the inner surface of the barrel, and/or suppress the reduction in cell viability.

Such a fluororesin can be fabricated by fluorination using a known method such as a method of performing stabilization by bringing the end group of a fluororesin synthesized according to a conventional method such as suspension polymerization or emulsion polymerization into contact with a fluorine-containing compound (for example, fluorine radical source) before melt extrusion of the fluororesin or a method of performing fluorination by bringing pellets of the fluororesin obtained into contact with a fluorine-containing compound after melt extrusion of the fluororesin. Further, a fluororesin can be obtained also by using a chain transfer agent or a polymerization catalyst that can control the end group together with fluorine monomers during production (polymerization reaction). Further, a commercially available product can be used as a fluororesin in the present invention. Further, fluorination can be performed by bringing a syringe that is a formed product formed by melting a fluororesin into contact with a fluorine-containing compound. Further, these treatment methods can be combined.

That is, the total of nonfluorinated group ends and - CF₂H group ends is not necessarily 70 or less per 1 × 10⁶ of carbon atoms at the stage of a fluororesin, pellets, and the like as a raw material and may be 70 or less per 1 × 10⁶ of carbon atoms on the surface in contact with the contents such as cells, nucleic acids, or proteins in the barrel of a syringe as a final product. Further, a fluororesin containing one or more -CF₃ end groups does not need to contain one or more -CF₃ end groups at the stage of a fluororesin, pellets, and the like as a raw material and the fluororesin may contain one or more -CF₃ end groups on the surface in contact with the contents such as cells, nucleic acids, or proteins in the barrel of a syringe as a final product.

The fluorine radical source is not specifically limited, but examples thereof can include halogen fluorides such as IF₅ and ClF₃, F₂ gas, CoF₃, AgF₂, UF₆, OF₂, N₂F₂, and CF₃OF. Such F₂ gas may have a concentration of 100% but is mixed with an inert gas to 5 to 50 mass%, preferably diluted to 15 to 30 mass% for use, in view of the safety. Examples of the inert gas can include nitrogen gas, helium gas, and argon gas, and nitrogen gas is preferable in view of the cost-effectiveness.

The fluorination is preferably performed at a temperature of 20 to 220°C, more preferably 100 to 200°C. The fluorination is preferably performed for 5 to 30 hours, more preferably 10 to 20 hours.

Examples of the polyethylene resin in the present invention include low density polyethylene (LDPE) resin, medium density polyethylene (MDPE) resin, high density polyethylene (HDPE) resin, ultrahigh density polyethylene resin, and ultrahigh molecular weight polyethylene (UHPE) resin.

In the present invention, the fluororesin or the polyethylene resin that can form the barrel, the gasket, the cap, the plunger, the finger grip, or the outer bag is preferably PTFE, FEP, PFA, LDPE, MDPE, HDPE, or UHPE for high impact resistance at very low temperatures. Further, in the case where the resin has a thickness to some extent, FEP, PFA, LDPE, MDPE, or HDPE is more preferable, and FEP or PFA is particularly preferable, so that the contents are easily visually recognized, in view of the transparency. Therefore, FEP or PFA is particularly preferable as a material of the barrel.

In the present invention, the barrel, the gasket, the cap, the plunger, the finger grip, or the outer bag may have a laminated structure or may not have a laminated structure, as long as the effects of the present invention are not impaired. In the case of forming a laminated structure using a plurality of layers made of different materials, the combination of materials is not particularly limited, as long as the shrinkage rates when cooled to a desired temperature are the same or within a close range, and the layers do not separate during cooling.

In the present invention, the volume of the barrel (volume of the syringe) is not particularly limited, and examples thereof include 0.5 mL or more, 1 mL or more, 2.5 mL or more,100 mL or less, 70 mL or less,50 mL or less, 30 mL or less, 20 mL or less, 15 mL or less, 10 mL or less, 5 mL or less, and 2.5 mL or less, that is 0.5 mL to 50 mL, 1 mL to 30 mL, 1 mL to 20 mL, 1 mL to 10 mL, 1 mL to 5 mL, 1 mL to 2.5 mL can be exemplified.

In the present invention, the inner diameter (A) of the barrel is not particularly limited, and examples thereof include 2 mm or more, 3 mm or more, 4 mm or more, 5 mm or more, 6 mm or more, 7 mm or more, 8 mm or more, 9 mm or more, 10 mm or more, 40 mm or less, 35 mm or less, 30 mm or less, 25 mm or less, 20 mm or less, 18 mm or less, 16 mm or less, 15 mm or less, 14 mm or less, 13 mm or less, 12 mm or less, 11 mm or less, and 10 mm or less, that is 2 mm to 40 mm, 3 mm to 30 mm, 4 mm to 20 mm, 4 mm to 18 mm, 5 mm to 18 mm, 5 mm to 15 mm, 5 mm to 12 mm, 6 mm to 10 mm can be exemplified.

In the present invention, the thickness of the barrel is not particularly limited, and examples thereof include 0.2 mm or more, 0.3 mm or more, 0.4 mm or more, 0.5 mm or more, 0.6 mm or more, 0.7 mm or more, 0.8 mm or more, 0.9 mm or more, 1.0 mm or more, 1.1 mm or more, 1.2 mm or more, 1.3 mm or more, 1.4 mm or more, 1.5 mm or more, 2.5 mm or less, 2.2 mm or less, 2.0 mm or less, 1.9 mm or less, 1.8 mm or less, 1.7 mm or less, 1.6 mm or less, 1.5 mm or less, 1.4 mm or less, 1.3 mm or less, 1.2 mm or less, 1.1 mm or less, 1.0 mm or less, 0.9 mm or less, 0.8 mm or less, 0.7 mm or less, 0.6 mm or less, and 0.5 mm or less, that is 0.2 mm to 2.5 mm, 0.3 mm to 2.2 mm, 0.4 mm to 2.0 mm, 0.3 mm to 1.9 mm, 0.4 mm to 1.8 mm, 0.5 mm to 1.7 mm, 0.6 mm to 1.6 mm, 0.7 mm to 1.5 mm, 0.4 mm to 1.2 mm, 0.5 mm to 1.1 mm, 0.6 mm to 1.0 mm, 0.7 mm to 0.9 mm can be exemplified. The resin forming the barrel preferably has a transparency to the extent that the contents such as cells stored in the barrel are easily visually recognized from the outside of the barrel, even in the case where the resin has a certain thickness. Here, examples of the transparency include a transmittance at a wavelength of 450 nm of 15% or more, 25% or more, 35% or more, 45% or more, and 55% or more.

In the present invention, the methods for producing the barrel and the gasket are not particularly limited, and they can be produced, for example, by general injection molding. The same applies to the plunger, the cap, and the finger grip, which will be described below.

The syringe of the present invention may further comprise a finger grip. The stability when using the syringe is enhanced to increase the usability by comprising the finger grip, since the plunger can be pressed with the thumb while fixing the syringe with the index finger and the middle finger on the finger grip when using the syringe. Here, the finger grip may be integrally formed with the barrel to be connected thereto. In this case, it is particularly preferable that it is formed from the same material as the material from which the barrel is formed, or may be detachable.

In the case where finger grip is detachable, it is preferable that the finger grip is not attached when the syringe is stored in the later-described outer bag, and the finger grip is attached when the syringe is taken out from the outer bag for use. The finger grip is not attached when the syringe is stored in the outer bag, thereby allowing the outer bag to easily adhere more closely to the syringe, so that vacuum packaging that does not allow a gas phase to exist between the outer bag and the syringe can be enabled. In the state of vacuum packaging that does not allow a gas phase to exist between the outer bag and the syringe, heat is directly conducted to the syringe without passing through the gas phase when the syringe stored in the outer bag is cooled and heated, and thus cooling and heating efficiency are improved, so that the time required for freezing and thawing the contents can be shortened. Therefore, particularly when cells or the like that are sensitive to the temperature change are stored in the syringe, quality deterioration and death of cells or the like due to a decrease in thawing speed can be suppressed.

Further, in the case where the finger grip is not detachable, it is preferable in the same manner when the outer circumferential surface of the finger grip and the outer circumferential surface of the barrel are partially flush or substantially flush with each other, since the outer bag easily adheres more closely to the syringe. Here, to be partially flush means to have a portion with no step between the outer circumferential surface of the finger grip and the outer circumferential surface of the barrel. To be substantially flush includes a state in which there is a step that does not hinder the adhesion of the outer bag to the syringe, other than the state of being flush. As an example of the syringe structure in which the outer circumferential surface of the finger grip and the outer circumferential surface of the barrel are partially flush or substantially flush with each other, the following aspect can be mentioned. That is, in the case where the cross section of the barrel is circular, and the finger grip is substantially elliptical, an aspect in which the diameter of the circle and the minor axis of the ellipse are the same or substantially the same can be mentioned as an example. Further, in the case where the cross section of the barrel is circular, and the finger grip is substantially rectangular, an aspect in which the diameter of the circle and the length of the short side of the rectangle is the same or substantially the same can be mentioned as an example.

Further, the syringe of the present invention may be stored in the outer bag. When the syringe is stored in the outer bag, the sanitary conditions during transportation, preservation, freezing, and thawing is maintained better, and the handling can be easier.

In the syringe of the present invention, the plunger is connected to the tip of the gasket, the user of the syringe pushes the plunger in the direction from the opening of the barrel toward the spout to move, and the gasket is configured to slide against the inner surface of the barrel. Therefore, the gasket and the plunger may be integrally formed, or the gasket and the plunger may be integrated by having a groove or a threaded structure for connecting them. In such a case, the plunger is preferably formed from the same resin as the resin forming the gasket. The aspect of the plunger is not particularly limited, as long as the function of sliding the gasket is not impaired, as mentioned above but when the gasket is slidably accommodated in the barrel, the plunger is preferably connected to the gasket with a gap from the barrel. At this time, it is more preferable that the plunger is connected to the gasket perpendicularly or substantially perpendicularly. The gap provided between the plunger and the barrel can eliminate or mitigate the resistance when the gasket slides in the barrel due to the contact between the plunger and the barrel, so that the gasket can slide better. The gap may be provided so that the plunger and the barrel are not in contact at all, or may be provided so that they are partially in contact, but it is preferably provided so that they are not in contact at all in view of the slidability, as mentioned above. The plunger is also generally referred to as a push rod.

The syringe of the present invention is a syringe specified for use "for cryopreservation" of a subject substance. Here, "cryopreservation" of the subject substance means to preserve the subject substance as the contents of the syringe in a frozen state for a certain period of time. Here, "preservation" is a state where the contents are not used, and for example, it can be a state where it is allowed to stand in a deep freezer, etc. or a state during transportation, etc. The preservation period is not particularly limited, but may be, for example, several minutes to several years. The contents of the syringe of the present invention are not particularly limited as long as they are substances to be cryopreserved, and examples thereof include substances for medical use, testing and research use, and the like. Also, the subject substance may be liquid, gas, solid, or a mixture thereof, and is not particularly limited as long as it is a substance that requires cryopreservation. Examples include cells, a nucleic acid, or a protein.

Since the syringe of the present invention can withstand preservation at very low temperatures, as mentioned above, suitable examples of the contents of the syringe of the present invention include cells, nucleic acids, and/or proteins that require cryopreservation at very low temperatures. Cells, nucleic acids, or proteins can be preserved stably over a long period of time by being frozen. Further, cells, nucleic acids, or proteins, especially when used for medical purposes, are stored in the syringe of the present invention in advance so that they can be directly administered to the subject of administration without being transferred to another container after cryopreservation and thawing. That is, the syringe of the present invention can be provided as a prefilled syringe storing contents (for example, cells, nucleic acids, or proteins). According to one aspect of the prefilled syringe, a syringe comprising the barrel, the gasket, the plunger, and, the cap, described above and storing contents, wherein the syringe is configured to detach the cap and attach the injection needle in use can be mentioned. Examples of the subject of administration can include mammals, amphibians, reptiles, birds, and fish, particularly suitably mammals. Examples of the mammals can include humans, pigs, cows, horses, goats, sheep, wild boars, rabbits, mice, rats, hamsters, guinea pigs, monkeys, rhesus monkeys, cynomolgus monkey, marmosets, orangutans, chimpanzees, dogs, and cats, suitably humans.

The temperature for cryopreservation in the present invention can be appropriately set according to the properties, physical properties, application, preservation purpose, preservation period, and the like of the contents, as long as the contents can be frozen and is not particularly limited but examples thereof include 0°C or less, -5°C or less, -10°C or less, -15°C or less, -20°C or less, -30°C or less, -40°C or less, -50°C or less, -60°C or less, -70°C or less, -80°C or less, -90°C or less, -100°C or less, -110°C or less, -120°C or less, -130°C or less, -140°C or less, - 150°C or less, -160°C or less, -170°C or less, -180°C or less, -190°C or less, -196°C or less, -200°C or less, -210°C or less, -220°C or less, -230°C or less, -240°C or less, and -250°C or less, and -300°C or more, -290°C or more, - 280°C or more, -273°C or more, -270°C or more, -260°C or more, -250°C or more, -240°C or more, -230°C or more, -220°C or more, -210°C or more, -200°C or more, -196°C or more, - 190°C or more, -180°C or more, -170°C or more, -160°C or more, -150°C or more, -140°C or more, -130°C or more, -120°C or more, -110°C or more, -100°C or more, -90°C or more, - 80°C or more, -70°C or more, -60°C or more, -50°C or more, -40°C or more, and -30°C or more, preferably -20°C or less, more preferably -80°C or less, for example -20°C to -250°C, -20°C to -196°C, -20°C to -150°C, -20°C to -130°C, -20°C to -100°C, -20°C to -80°C, -150°C to -250°C, -150°C to -196°C, and -150°C to -180°C.

In addition, examples of the temperature for cryopreservation, especially when the contents are cells, nucleic acids, or proteins, include -20°C or less, -30°C or less, -40°C or less, -50°C or less, -60°C or less, -70°C or less, -80°C or less, -90°C or less, -100°C or less, -110°C or less, -120°C or less, -130°C or less, -140°C or less, - 150°C or less, -160°C or less, -170°C or less, -180°C or less, -190°C or less, and -196°C or less, and -200°C or more, -196°C or more, -190°C or more, -180°C or more, -170°C or more, -160°C or more, -150°C or more, -140°C or more, -130°C or more, -120°C or more, -110°C or more, -100°C or more, - 90°C or more, -80°C or more, -70°C or more, -60°C or more, -50°C or more, -40°C or more, and -30°C or more, preferably -80°C or less, for example, -80°C to -196°C, -80°C to - 180°C, -80°C to -150°C, -80°C to -130°C, -80°C to -100°C, - 150°C to -196°C and -150°C to -180°C. In general, it is said that the lower the cryopreservation temperature, the better the long-term preservation stability, and when it is -196°C or less, semi-permanent preservation is possible. Cells in particular may be preserved at about -20°C for a short period of time and then preserved at -80°C or less for a long period of time. Although the method of freezing cells, nucleic acids, or proteins is not particularly limited, examples thereof include a freezing method using a deep freezer with a cooling capacity of -80°C or less, and a freezing method using the gas phase (-130°C or less) or liquid phase (-196°C) of liquid nitrogen.

The syringe of the present invention can be a syringe that is used at the above-mentioned temperature for cryopreservation, and examples include to be a syringe that is used at -80°C or less for cryopreservation, a syringe that is used at -150°C or less for cryopreservation. That is, the syringe of the present invention can be a syringe that is for use in the cryopreservation at the above-mentioned temperature, and examples include to be a syringe that is for use in the cryopreservation at -80°C or less, a syringe that is for use in the cryopreservation at -150°C or less. It is the same for the prefilled syringe of the present invention.

Further, the syringe of the present invention can be used by cooling to the above temperature to freeze the contents and then heating to thaw the contents. The thawing temperature is not particularly limited but examples thereof include -20°C or more, -10°C or more, 0°C or more, 10°C or more, 20°C or more, 30°C or more, 37°C or more, 40°C or more, 50°C or more, 60°C or more, 70°C or more, 80°C or more, and 100°C or less, 80°C or less, 60°C or less, 40°C or less, 20°C or less, and 0°C to 60°C. Especially when the contents are cells, nucleic acids, or proteins, examples thereof include -20°C or more, -10°C or more, 0°C or more, 10°C or more, 20°C or more, 30°C or more, and 37°C or more, 40°C or less, and room temperature, for example, 0°C to 40°C, 20°C to 40°C, and 30°C to 40°C. The thawing method is not particularly limited but examples thereof include a thawing method gently on ice, or a thawing method by setting the desired temperature using a heating device such as a hot water bath.

In the present invention, examples of the cells include mammalian cells, and the mammalian cells may be buoyant cells or adherent cells. Examples of the buoyant cells can include erythrocytes, (peripheral blood-derived) leukocytes (neutrophils, mononuclear cells [monocytes, lymphocytes], macrophages). Examples of adherent cells include stem cells including pluripotent stem cells such as embryonic stem cells (ES cells), embryonic germ cells (EG cells), germline stem cells (GS cells), and induced pluripotent stem cells (iPS cells), multipotent stem cells such as mesenchymal stem cells, hematopoietic stem cells, and nervous system stem cells, unipotent stem cells (progenitor cells) such as cardiac progenitor cells, vascular endothelial progenitor cells, neural progenitor cells, adipose progenitor cells, dermal fibroblasts, skeletal muscle myoblasts, osteoblasts, and odontoblasts, mature cells including cardiomyocytes, vascular endothelial cells, nerve cells, adipocytes, dermal fiber cells, skeletal muscle cells, osteocytes, hepatocyte (liver) cells, umbilical vein endothelial cell, dermal microlymphatic endothelial cells, epidermal keratinocytes, bronchial epithelial cells, melanocytes, smooth muscle cells, and dentin cells. Examples of the application of cells include medical uses and testing and research uses, and cells such as stem cells (therapeutic cells) for use in medical treatments such as regenerative medicine are particularly suitable. The cells may be suspended in a cell culture medium, cell preservative solution, or the like.

In the present invention, the nucleic acids are not particularly limited, as long as they are natural, modified, or synthetic nucleotides, and may be DNA, RNA, or chimeras thereof. Generally, a nucleic acid is a gene or an analogue thereof, more specifically the gene is, for example, a tumor suppressor gene, and more specifically a polynucleotide or an oligonucleotide as a gene analogue. Here, nucleotides include oligonucleotides modified to make them less susceptible to degradation in vivo such as an oligonucleotide having a thiophosphodiester bond (S-oligo) in which the oxygen atom of the phosphodiester bond is replaced with a sulfur atom, or an oligonucleotide in which the phosphodiester bond is replaced with an uncharged methylphosphate group. Further specifically, examples of the oligonucleotide include decoys (decoy molecules), antisense, ribozymes, aptamers, or siRNA or mRNA. Examples of the application of nucleic acids include medical uses and testing and research uses, and these nucleic acids as pharmaceuticals are particularly preferable. Here, pharmaceuticals include RNA vaccines. Nucleic acids may be in a lyophilized (freeze-dried) state, or may be in a state of being suspended or dissolved in a nucleic acid preservation solution or the like.

In the present invention, proteins refer to single or multiple polymer compounds formed by linking (polymerizing) a large number of L-amino acids in a chain via amide bonds (also called peptide bonds) and are not limited by the number of constituent amino acids. Accordingly, the proteins of the present invention also include so-called peptides. Further, the proteins of the present invention also include glycoproteins in which sugars and proteins are bound and lipoproteins in which lipids and proteins are bound. Examples of the proteins include cell growth factors such as albumin, fibrinogen, globulin (α1-globulin, α2-globulin, β-globulin, and γ-globulin), erythropoietin, collagen, elastin, keratin, lactoferrin, avidin, cadherin, proteoglycan, mucin, LDL (Low Density Lipoprotein), HDL (High Density Lipoprotein), VLDL (Very Low Density Lipoprotein), insulin, and transferrin, cytokines and growth factors such as activin A, Bone Morphogenetic Proteins 4 (BMP-4), Epidermal Growth Factor (EGF), Stem Cell Factor (SCF), interleukin. Examples of the application of proteins include medical uses and testing and research uses, and these proteins as pharmaceuticals are particularly preferable. The proteins may be in a lyophilized (freeze-dried) state, or may be in a state of being suspended or dissolved in a protein preservation solution or the like.

The purpose of use of the syringe of the present invention is not particularly limited, but the syringe of the present invention can be a syringe that is used for medical procedure (regenerative medicine, dosing, vaccination, etc.), manufacture of medicine, research, or the like. Examples of regenerative medicine include, administering therapeutic cells such as stem cells to a patient, going through processes such as collecting, selecting, concentrating, and/or proliferation, according to the purpose. The syringe of the present invention can be cryopreserved one or more times when using, according to the purpose.

The specific method of use of the syringe of the present invention is not particularly limited, but the syringe of the present invention can be used for example in a method for cryopreserving a subject substance, as in the following.
(1) providing a subject substance to be cryopreserved (that should be cryopreserved);
(2) providing a syringe comprising: a barrel having a spout and an opening; a gasket slidably accommodated in the barrel, a plunger connected to the gasket; and a cap that seals the spout of the barrel, wherein the opening of the barrel is sealed only with the gasket;
(3) pulling to move the gasket by an operation of the plunger to the direction from the spout to the opening of the barrel to store the subject substance in the barrel;
(4) attaching the cap to the spout of the barrel to seal the barrel;
(5) storing the syringe in the outer bag if necessary, and further close or seal the outer bag if necessary;
(6) putting the syringe under a desired low temperature (for example, at -20°C or less, -80°C or less, or -150°C or less) to freeze the subject substance in the syringe to preserve for a desired period;

Further, the syringe of the present invention can be used for example in a method for administering a subject substance, as in the following.
(1) providing a subject substance to be administered (that should be administered);
(2) providing a syringe comprising: a barrel having a spout and an opening; a gasket slidably accommodated in the barrel, a plunger connected to the gasket; and a cap that seals the spout of the barrel, wherein the barrel is formed from a fluororesin, and the opening of the barrel is sealed only with the gasket;
(3) pulling to move the gasket by an operation of the plunger to the direction from the spout to the opening of the barrel to store the subject substance in the barrel;
(4) attaching the cap to the spout of the barrel to seal the barrel;
(5) storing the syringe in the outer bag if necessary, and to further close or seal the outer bag if necessary;
(6) putting the syringe under a desired low temperature (for example, at -80°C or less, or -150°C or less) to freeze the subject substance in the syringe to preserve for a desired period;
(7) putting the syringe under a desired high temperature (for example, at room temperature, 30°C or more, or 37°C) to thaw the subject substance for a desired period;
(8) taking out the syringe from the outer bag in case the syringe is stored in the outer bag;
(9) detaching the cap from the spout of the barrel of the syringe, and attaching the injection needle to the spout;
(10) administering the subject substance from the syringe to a subject in need of administration of the subject substance.

The syringe or prefilled syringe of the present invention may be provided by being contained in a kit. As kits, examples include a kit comprising the syringe or prefilled syringe of the present invention, and an instruction for use describing the method for cryopreserving or administering a subject substance using the syringe or prefilled syringe of the present invention (i.e. for performing the method). The kit of the present invention can be a kit that is used for cryopreserving and/or administering a subject substance. The kit of the present invention may further contain other constituents. Examples of other constituents include a cryopreservation solution for cryopreserving a subject substance, a dissolution buffer, a carrier, a pH buffer, a stabilizer, an instruction manual, other attached documents, an injection needle, a tube, etc.

### Examples

Hereinafter, the present invention will be described further specifically by way of examples, but the technical scope of the present invention is not limited to these examples.

### 1. Analytical evaluation of fluororesin

It is known that a fluororesin in which a total number of non-fluorinated group ends and -CF₂H group ends in the fluororesin is 70 or less per 1 × 10⁶ of carbon atoms, and/or containing one or more -CF₃ end groups can suppress adsorption of cells (i.e. can suppress decrease in cell recovery rate), can suppress decrease in cell viability, and further can suppress adsorption of protein (see WO2017/010100 and WO2018/135228). Therefore, resin Q and resin R shown in Table 1 were used for producing syringes. For both resin Q and resin R, in the sample before molding syringes (pellet), the number of non-fluorinated group ends and the number of -CF₂H group ends were both 0 per 1 × 10⁶ of carbon atoms. Further, in the sample after molding syringes of resin R, the number of non-fluorinated group ends was 48 to 66 per 1 × 10⁶ of carbon atoms, and the number of -CF₂H group ends is 0 per 1 × 10⁶ of carbon atoms as shown in Table 1, that is the total number of non-fluorinated group ends and -CF₂H group ends was 70 or less per 1 × 10⁶ of carbon atoms. Here, since similar syringe molding processing as for resin R is performed for resin Q, it is estimated that the change in the number of non-fluorinated group ends and the number of -CF₂H group ends after syringe molding has the same tendency. As such, it is inferred that in the sample after molding syringes of resin Q, the total number of non-fluorinated group ends and -CF₂H group ends is 70 or less per 1 × 10⁶ of carbon atoms, and the number of -CF₂H group ends is 0 per 1 × 10⁶ of carbon atoms. The number of non-fluorinated group ends and -CF₂H group ends was measured according to the method described in WO2017/010100 and WO2018/135228.

**Table 1: Analytical evaluation of fluororesin**

| | | Sample after molding syringe | | |
|---|---|---|---|---|
| Resin name | Materi al of resin | number of non-fluorinated group ends (per 1 × 10⁶ of carbon atoms) | number of -CF₂H group ends (per 1 × 10⁶ of carbon atoms) | Presence or absence of -CF₃ end groups |
| Resin Q | FEP | (no data) | (no data) | Present |
| Resin R | PFA | 1.0 mL volume syringe: 48 | 1.0 mL volume syringe: 0 | 1.0 mL volume syringe: present |
| | | 2.5 mL volume syringe: 66 | 2.5 mL volume syringe:0 | 2.5 mL volume syringe: present |

| | | | | |
|---|---|---|---|---|
| Non-fluorinated group ends: -COF, -COOH, -COOH associated with water, -CH₂OH, - CONH₂, and -COOCH₃ | | | | |

### 2. Evaluation of durability during cooling (1): Tensile impact test

In order to evaluate the impact resistance during cooling of each material, a tensile impact test was conducted.

### 2-1 Method

### 2-1-1 Test sample

Each resin shown in Table 2 was subjected to the test.

### 2-1-2 Test operation

[1] A test piece obtained by molding each resin into the shape of type 2 of JIS K 7160, a crosshead, and a fixing jig were cooled over about 1 minute in liquid nitrogen.
[2] The test piece, the crosshead, and the fixing jig cooled in [1] above were attached to a tensile impact tester (fully automatic Charpy impact tester, No. 190, manufactured by YASUDA SEIKI SEISAKUSHO, LTD.).
[3] Liquid nitrogen was spun around the test piece attached to the tester to cool it again.
[4] The test was conducted immediately after [3] above. This test was performed three times for the same resin, and the average and standard deviation were obtained. The weights of the crosshead hammer and the hammer were appropriately adjusted according to the test piece and measurement conditions.

### 2-2 Results

Table 2 shows the test results. The results indicate that the higher the absorbed energy (J), the higher the impact resistance. It was shown that fluororesin and PE resin have high impact resistance under liquid nitrogen cooling conditions, and among them, UHPE has extremely high impact resistance under liquid nitrogen cooling conditions. It was also shown that among fluororesins, PTFE in particular has high impact resistance under liquid nitrogen cooling conditions. Meanwhile, PP resin was shown to have low impact resistance under liquid nitrogen cooling conditions as compared to the above. As can be seen from the above, it was shown that fluororesin or PE resin are suitable materials for syringes that are assumed to be used under liquid nitrogen cooling conditions.

**Table 2: Absorption energy**

| Resin sample No. | Material of resin | Absorption energy (J) |
|---|---|---|
| 1 | FEP | 0.34±0.08 |
| 2 | PFA | 0.32±0.06 |
| 3 | PTFE | 0.78±0.13 |
| 4 | UHPE | 2.99±0.14 |
| 5 | HDPE | 0.40±0.02 |
| 6 | LDPE | 0.37±0.13 |
| 7 | PP | 0.22±0.04 |

| | | |
|---|---|---|
| Abbreviations in the table indicate the following resins. FEP: Resin Q in Table 1 PFA: Resin R in Table 1 PTFE: Manufactured by Flonchemical, Co., Ltd. UHPE: Newlight, manufactured by Shimonoseki Packing, Co., Ltd. HDPE: Manufactured by Flonchemical, Co., Ltd. LDPE: Manufactured by Flonchemical, Co., Ltd. PP: Manufactured by Flonchemical, Co., Ltd. | | |

### 3. Evaluation of durability during cooling (2) Drop test

In order to evaluate the embrittlement resistance of a syringe formed from each material during cooling, a drop test was conducted.

### 3-1 Method

### 3-1-1 Test sample

The 1.0 mL volume syringes of syringe sample Nos. 7-1 to 7-3 each consisting of a barrel, a gasket, a plunger, a finger grip, and a cap (see Table 3) were subjected to the test. The cap was common to all syringes, and a combination cap (product number: 4495209 made of PE, manufactured by B. Braun Melsungen AG) was used.

### 3-1-2 Test operation

[1] Four syringes were prepared for each, and each was filled with 1.0 mL of water and capped.
[2] The syringes of [1] above were put into a deep freezer (-80°C) and preserved for one day or more.
[3] The syringes were taken out from the deep freezer and dropped onto an iron plate from a height of 120 cm.
[4] The syringes dropped in [3] above were observed with the cap detached, to confirm the presence or absence of damage.

### 3-2 Results

Table 3 shows the test results. One of the four syringes 7-2 subjected to the test was damaged by cracking at the spout of the barrel. Syringe 7-2 was a syringe with a barrel formed from PP resin. PP resin generally has a glass transition temperature of about 0°C to -10°C, that is, it is known to be embrittled at a low temperature below it, and this test also shows that PP resin is unsuitable as a material for syringes that are assumed to be cryopreserved at -80°C or less.

Meanwhile, syringe 7-1 having a barrel and a gasket formed from PFA resin was not damaged at all even when dropped onto an iron plate from a height of 120 cm and was not damaged when cooled in the gas phase of liquid nitrogen (-150°C or less) and thereafter hit against the iron plate. This result showed that PFA resin is highly suitable as a material for forming a syringe that is assumed to be cryopreserved at -80°C or less, further at -150°C or less.

**Table 3: The number of damaged syringes**

| Syringe sample No. | Barrel material | Gasket material | The number of damaged syringes |
|---|---|---|---|
| 7-1 | PFA | PFA | 0 |
| 7-2 | PP | Thermoplastic elastomer | 1 |
| 7-3 | PP | PE | 0 |

| | | | |
|---|---|---|---|
| n=4. The terms in the table indicate the following resins. PFA: Resin R in Table 1 7-1: Prototype manufactured by injection molding, Volume: 1.0 mL 7-2: Manufactured by JMS Co., Ltd. (Model:JS-SM01C), Volume: 1.0 mL 7-3: Manufactured by Henke-Sass, Wolf GmbH (Code: S4010-LT), Volume: 1.0 mL | | | |

### 4. Evaluation of shrinkage rate during cooling

In order to evaluate the shrinkage rate of each material during cooling, the following test was conducted.

### 4-1 Method

### 4-1-1 Test sample

The resins (or elastomers) shown in Table 4 were subjected to the test.

### 4-1-2 Test operation

[1] A disc-shaped resin piece having a diameter of 5 mm and a thickness of 1 mm was fabricated for each resin.
[2] The resin piece was left standing at room temperature and then the diameter (a) was measured.
[3] The resin piece was cooled using a microscope cooling and heating stage at 30°C/minute, followed by standing at - 130°C for 20 minutes, and the diameter (b) was measured.
[4] The shrinkage rate of the resin during cooling (-130°C) was determined by the following calculation formula. Calculation formula: Shrinkage rate (%) = {(a - b)/a} × 100

### 4-2 Results

Table 4 shows the test results. All of the resins were shown to shrink more when cooled at -130°C than at room temperature, but the magnitude of the shrinkage rate significantly differed among the resins as shown in Table 4.

**Table 4: Shrinkage rate during cooling at low temperature (-130°C)**

| Resin sample No. | Material of resin | Shrinkage rate (%) |
|---|---|---|
| 8-1 | PP | 1.2 |
| 8-2 | PE | 1.6 |
| 8-3 | Silicone | 3.8 |
| 8-4 | SEBS | 0.8 |
| 8-5 | FEP | 2.0 |
| 8-6 | PFA | 1.6 |

| | | |
|---|---|---|
| n=1. The terms in the table indicate the following resins. SEBS: SEBE (styrene ethylene butylene styrene; thermoplastic elastomer) cut from the gasket of Terumo Syringe, manufactured by Terumo Corporation FEP: Resin Q in Table 1 PFA: Resin R in Table 1 | | |

On the assumption that the barrel and the gasket of a syringe are formed by combining these resins, Table 5 shows a value calculated as a ratio (X/Y) of a shrinkage rate (X) of a resin forming the barrel during cooling at a low temperature (-130°C) to a shrinkage rate (Y) of a resin forming the gasket during cooling at a low temperature (-130°C) in each case. The value of Table 4 was used for calculation.

Theoretically, a gap occurs between the barrel and the gasket when (Y) is larger than (X), and therefore the integrity of the syringe cannot be maintained. Therefore, in order to form a syringe that is assumed to be cryopreserved, 1 ≤ (X/Y) is considered necessary to be satisfied. Further, it is considered that there is a fear that the gasket or the barrel may be damaged when the ratio (X/Y) is excessively large, since it cannot withstand the shrinkage pressure of the barrel during cooling at low temperature, or the gasket may be disengaged from the barrel. However, as shown in Table 5, the ratio (X/Y) was expected to range very widely from 0.32 to 2.50 depending on the combination of resins. As can be seen from the above, it was suggested that it was important to consider that the ratio of the shrinkage rate of each resin during cooling at low temperature satisfies 1 ≤ (X/Y), and the ratio (X/Y) falls within the range comparatively close to 1 in the combination of the materials as resins forming the barrel and the gasket of a syringe that is assumed to be preserved at very low temperatures.

**Table 5: Ratio (X/Y) of shrinkage rate (X) of resin forming barrel during cooling at low temperature (-130°C) to shrinkage rate (Y) of resin forming gasket during cooling at low temperature (-130°C)**

| | | Material of resin forming gasket | | | | | |
|---|---|---|---|---|---|---|---|
| | | PP | PE | Silicone | SEBS | FEP | PFA |
| Material of resin forming barrel | PP | 1.00 | 0.75 | 0.32 | 1.50 | 0.60 | 0.75 |
| | PE | 1.33 | 1.00 | 0.42 | 2.00 | 0.80 | 1.00 |
| | FEP | 1.67 | 1.25 | 0.53 | 2.50 | 1.00 | 1.25 |
| | PFA | 1.33 | 1.00 | 0.42 | 2.00 | 0.80 | 1.00 |

| | | | | | | | |
|---|---|---|---|---|---|---|---|
| *n=1. Each value was determined from value shown in Table 4. The terms in the table indicate the following resins. SEBS: SEBE cut from the gasket of Terumo Syringe, manufactured by Terumo Corporation FEP: Resin Q in Table 1 PFA: Resin R in Table 1 | | | | | | | |

### 5. Evaluation of slidability of syringe, and evaluation of integrity during cooling and heating (1)

In order to determine whether or not a syringe formed from each material can withstand a practical use for cryopreservation at -80°C by evaluating the slidability of the syringe, and the integrity during cooling and heating, the following tests for slidability and integrity were conducted.

### 5-1 Method

### 5-1-1 Test sample

The syringes of sample Nos. 9-1-1 to 9-6-4 shown in Table 6 were produced by injection molding and subjected to the tests. These were 1.0 mL volume syringes each consisting of a barrel, a gasket composed of one gasket lip, a plunger, and a finger grip. The gasket had a discotic shape with uniform thickness, and the shape of the sliding contact portion with the inner surface of the barrel was a flat surface extending in a sliding contact direction. Further, as comparative controls, commercially available syringes (syringe sample Nos. 9-7 to 9-8) were also subjected to the tests (see Table 8). All these syringes subjected to the tests had an integrity at room temperature. Further, the cap used in the integrity test was common to all syringes, and a combination cap (product number: 4495209 made of polyethylene, manufactured by B. Braun Melsungen AG) was used. The cap had a shape including a substrate and a mantle tube fitted from a circumferential edge of the substrate to an outer circumferential surface of the spout of the barrel.

### 5-1-2 Test operation: Slidability test

[1] Using a tensile tester (TENSILON, RTG-1225, manufactured by A&D Company, Limited), the maximum load (N) at a moving distance of 40 mm at a moving speed of 100 mm/minute was measured. The measurement was performed 3 times with respect to the same syringe, and the average was obtained.
[2] The slidability of the syringe was determined to be good (Good) when the maximum load was 8 N or less and poor (Poor) when it was greater than 8 N.

### 5-1-3 Test operation: Integrity test

[1] A yellow pigment was dissolved in 99.5% ethanol to fabricate a colored ethanol (freezing point: about -120°C). The colored ethanol was filled into a PE bag.
[2] The gasket was pulled to the display volume of the syringe, the cap was attached without anything stored in the barrel, the syringe was immersed in the colored ethanol in the PE bag, and the PE bag was closed.
[3] The PE bag was put into a deep freezer (-80°C) and preserved for 3 to 7 days.
[4] The PE bag was taken out from the deep freezer and heated in a hot water bath at 37°C for 5 minutes or more.
[5] The syringe was taken out from the PE bag and lightly washed with water, and then the water was wiped off.
[6] The presence or absence of the intrusion of the colored ethanol into the barrel of the syringe was visually observed. For each syringe, when the intrusion of the colored ethanol was confirmed, it was determined to have no integrity (Poor), and when the intrusion of the colored ethanol was not confirmed, it was determined to have integrity (Good).

### 5-2 Results

Table 6 shows the test results. The data in Table 6 are sorted so that the ratio (B/A) is in ascending order. Even by sealing the opening of the barrel of the syringe only with the gasket, a syringe with good slidability in which the integrity can be maintained not only at room temperature, but also during any of cooling from room temperature to a very low temperature such as -80°C, preservation at the temperature, and heating from the temperature to 37°C could be obtained.

In the preliminary consideration, when inserting a gasket made of HDPE with a diameter (B) of about 7 mm to a barrel made of PFA with an inner diameter (A) of 6.37 mm (B/A = about 1.100), it was felt that the slidability was not good. Therefore, in the present test, a gasket having a smaller diameter (B) than the above was produced, and subjected to the test.

Further, in the syringe shown in Table 6, the thickness of the barrel of the currently designed syringe was in the range of 0.72 to 0.94 mm, and the slidability was not significantly affected by the thickness of the barrel in such a range.

Further, the average of the maximum load (N) of each gasket material was calculated from the data shown in Table 6 (see Table 7). As a result, the average of the maximum load (N) was smaller when a gasket made of a PTFE, UHPE, HDPE, or LDPE material was combined with a barrel made of a PFA material than when a gasket made of a PFA material was combined. This fact suggested that it is more preferable to combine a gasket made of PTFE, UHPE, HDPE, or LDPE material that is different from a barrel made of PFA material than a combination of a gasket made of the same PFA material with the barrel, particularly in view of slidability.

Meanwhile, it was shown that commercially available syringes shown in Table 16 could not be used for cryopreservation since the integrity is lost during any of cooling from room temperature to -80°C, preservation at the temperature, or heating from the temperature to 37°C.

It was shown from the above that according to the present invention, in a syringe formed by from a barrel, a gasket, a plunger, and a cap, a syringe that can be used for cryopreservation with good slidability in which the integrity can be maintained not only at room temperature but also during cold preservation at a very low temperature of such as -80°C and heating, and the usability and the safety are excellent can be provided without providing a special structure such as a cap or a seal at the barrel opening or without using a silicone compound or a rubber-based material, even by sealing the opening of the barrel only with the gasket.

**Table 6: Evaluation of slidability and evaluation of integrity during cooling and heating**

| | Barrel | | Gasket | | | Ratio | | Slidability | | Integrity |
|---|---|---|---|---|---|---|---|---|---|---|
| Syringe sample No. | Material | Inner diameter (A) (mm) | Material | Diameter (B) (mm) | Thickness (C) (mm) | B/A | C/B | Maximum load (N) | Evaluation | Evaluation |
| 9-1-2 | PFA | 6.37 | PFA | 6.37 | 1.0 | 1000 | 0.157 | 18.2 | Poor | Good |
| 9-1-3 | PFA | 6.37 | PFA | 6.38 | 1.0 | 1.002 | 0.157 | 18.5 | Poor | Good |
| 9-1-4 | PFA | 6.37 | PFA | 6.38 | 1.0 | 1.002 | 0.157 | 20.9 | Poor | Good |
| 9-6-2 | PFA | 6.37 | LDPE | 6.38 | 0.5 | 1.002 | 0.078 | 3.7 | Good | Good |
| 9-1-1 | PFA | 6.37 | PFA | 6.40 | 1.0 | 1.005 | 0.156 | 18.4 | Poor | Good |
| 9-6-1 | PFA | 6.37 | LDPE | 640 | 0.5 | 1.005 | 0.078 | 3.4 | Good | Good |
| 9-2-1 | PFA | 6.38 | PFA | 6.42 | 0.5 | 1.006 | 0.078 | 6.7 | Good | Good |
| 9-3-2 | PFA | 6.37 | PTFE | 6.42 | 0.5 | 1.008 | 0.078 | 6.2 | Good | Good |
| 9-3-3 | PFA | 6.37 | PTFE | 6.42 | 0.5 | 1.008 | 0.078 | 5.7 | Good | Good |
| 9-3-4 | PFA | 6.37 | PTFE | 6.42 | 0.5 | 1.008 | 0.078 | 4.6 | Good | Good |
| 9-6-3 | PFA | 6.37 | LDPE | 6.42 | 0.5 | 1.008 | 0.078 | 3.9 | Good | Good |
| 9-2-2 | PFA | 6.37 | PFA | 6.43 | 0.5 | 1.009 | 0.078 | 7.6 | Good | Good |
| 9-3-1 | PFA | 6.37 | PTFE | 6.43 | 0.5 | 1.009 | 0.078 | 6.1 | Good | Good |
| 9-4-2 | PFA | 6.37 | UHPE | 6.44 | 0.5 | 1.011 | 0.078 | 6.1 | Good | Good |
| 9-4-5 | PFA | 6.37 | UHPE | 6.44 | 0.5 | 1.011 | 0.078 | 5.7 | Good | Good |
| 9-4-1 | PFA | 6.37 | UHPE | 6.45 | 0.5 | 1.013 | 0.078 | 5.3 | Good | Good |
| 9-4-4 | PFA | 6.37 | UHPE | 6.47 | 0.5 | 1.016 | 0.077 | 4.2 | Good | Good |
| 9-6-4 | PFA | 6.37 | LDPE | 6.47 | 0.5 | 1.016 | 0.077 | 4.7 | Good | Good |
| 9-5-1 | PFA | 6.37 | HDPE | 6.49 | 0.5 | 1.019 | 0.077 | 6.0 | Good | Good |
| 9-5-5 | PFA | 6.37 | HDPE | 6.49 | 0.5 | 1.019 | 0.077 | 6.4 | Good | Good |
| 9-5-3 | PFA | 6.37 | HDPE | 650 | 0.5 | 1.020 | 0.077 | 6.7 | Good | Good |
| 9-5-4 | PFA | 6.37 | HDPE | 650 | 0.5 | 1.020 | 0.077 | 5.9 | Good | Good |
| 9-5-2 | PFA | 6.37 | HDPE | 6.51 | 0.5 | 1.022 | 0.077 | 6.9 | Good | Good |
| 9-4-3 | PFA | 6.37 | UHPE | 6.52 | 0.5 | 1.024 | 0.077 | 5.9 | Good | Good |

| | | | | | | | | | | |
|---|---|---|---|---|---|---|---|---|---|---|
| Preservation period in deep freezer was 3 days in the evaluation of integrity. The terms in the table indicate the following resins. PFA: Resin R in Table 1 UHPE: Newlight, manufactured by Simonoseki Packing, Co., Ltd. HDPE: Manufactured by Flonchemical, Co., Ltd. LDPE: Manufactured by Flonchemical, Co., Ltd. | | | | | | | | | | |

**Table 7: Average of maximum load (N) for each gasket material when evaluating slidability**

| Syringe sample No. | Barrel material | Gasket material | Average of maximum load (N) |
|---|---|---|---|
| 9-2-1 to 2(n=2) | PFA | PFA | 7.2 |
| 9-3-1 to 4(n=4) | PFA | PTFE | 5.7 |
| 9-4-1 to 5(n=5) | PFA | UHPE | 5.4 |
| 9-5-1 to 5(n=5) | PFA | HDPE | 6.4 |
| 9-6-1 to 4(n=4) | PFA | LDPE | 3.9 |

| | | | |
|---|---|---|---|
| It was calculated based on data of syringe having 0.5 mm thick gasket shown in Table 6. | | | |

**Table 8: Evaluation of integrity during cooling and heating of commercially available syringe**

| Syringe Sample No. | Barrel material | Gasket material | Determination of integrity |
|---|---|---|---|
| 9-7 | PP | Thermoplastic elastomer | Poor |
| 9-8 | PP | Thermoplastic elastomer | Poor |

| | | | |
|---|---|---|---|
| n=4. Storage period in deep freezer was 7 days. The numbers in the table indicate the following syringes. 9-7: Manufactured by Terumo Corporation (Code No.: SS-02LZ), Volume: 2.5 mL, Thermoplastic elastomer was SEBS, containing silicone oil as lubricant. 9-8: Manufactured by JMS Co., Ltd. (Model: JS-SM01C), Volume: 1.0 mL | | | |

### 6. Studies on shape of finger grip

In order to evaluate how the shape of the finger grip of the syringe affects the syringe when preserved while being stored in the outer bag, the following test was conducted.

### 6-1 Method

### 6-1-1 Test sample

A commercially available syringe (manufactured by NIPRO CORPORATION, volume: 2.5 mL) was used. The syringe had a general shape consisting of a barrel, a gasket, a plunger, and a finger grip, wherein the finger grip had a substantially elliptical shape, but there was no portion without steps between the outer circumferential surface of the finger grip and the outer circumferential surface of the barrel (that is, there was no portion flush with each other). Syringes in which finger grips having such a shape were processed into a substantially elliptical shape so that there were two portions without steps between the outer circumferential surface of the finger grip and the outer circumferential surface of the barrel (that is, there were two portions flush with each other) were referred to as "processed group". Meanwhile, syringes that were not subjected to such processing were referred to as "unprocessed group".

### 6-1-2 Test operation

[1] Three syringes from each of the processed group and the unprocessed group (syringe sample Nos. 10-1-1 to 10-2-3) were each filled with 2.5 mL of an aqueous solution (aqueous solution containing 10% DMSO and 0.81% NaCl) imitating a cell cryopreservation solution and capped (combination cap, product number: 4495209, manufactured by B. Braun Melsungen AG) .
[2] Each syringe was packaged one by one with an outer bag made of PE, closed with the air removed from the outer bag, and stored in a deep freezer (-80°C), to completely freeze the aqueous solution in the syringe.
[3] Each syringe was taken out from the deep freezer and immersed in a hot water bath at 37°C without opening the outer bag, to start thawing. 2 minutes, 4 minutes, and 6 minutes after thawing was started, the syringe was visually observed, and it was determined to be in any state in which the aqueous solution in the syringe was completely thawed (Good) or not completely thawed (Poor). At the time when the aqueous solution in the syringe completely thawed the observation of the syringe was ended.

### 6-2 Results

Table 9 shows the test results. As compared with that in the syringes of the unprocessed group, the aqueous solution in the syringes of the processed group could be completely thawed in a comparatively short time. This is thought to be because the outer circumferential surface of the finger grip and the outer circumferential surface of the barrel were partially flush with each other in the syringes of the processed group, and thus the outer bag easily adheres closely to the syringe, that is, the gas phase existing between the outer bag and the syringe could be reduced than in the unprocessed group, thereby allowing the heat to be conducted directly to the syringe without passing through the gas phase during thawing and improving the heating efficiency. In this case, it is considered that not only the heating efficiency but also the cooling efficiency would be improved. Therefore, the aforementioned results show that, in syringes that are assumed to be cryopreserved and thawed, the outer circumferential surface of the finger grip and the outer circumferential surface of the barrel are preferably partially flush with each other.

**Table 9: Evaluation of thawing time required**

| | | Elapsed time after starting thawing | | |
|---|---|---|---|---|
| | Syringe sample No. | 2 minutes | 4 minutes | 6 minutes |
| Processed group | 10-1-1 | Poor | Good | - |
| | 10-1-2 | Poor | Poor | Good |
| | 10-1-3 | Poor | Poor | Good |
| Unprocessed group | 10-2-1 | Poor | Good | - |
| | 10-2-2 | Poor | Poor | Poor |
| | 10-2-3 | Poor | Poor | Poor |

| | | | | |
|---|---|---|---|---|
| Good: Aqueous solution in syringe was completely thawed. Poor: Aqueous solution in syringe was not completely thawed. | | | | |

### 7. Evaluation of slidability of syringe, and evaluation of integrity during cooling and heating (2)

In order to determine whether or not the syringe of the present invention can withstand a practical use for cryopreservation at -150°C by evaluating the slidability of the syringe, and the integrity during cooling and heating, the following tests for slidability and integrity were conducted.

### 7-1-1 Test sample

The barrels and gaskets shown in Table 10 were produced by injection molding and subjected to the tests. These were 2.5 mL volume syringes each consisting of a barrel, a gasket composed of one gasket lip, a plunger, and a finger grip. The gasket had a discotic shape with uniform thickness of 0.31 mm, and the shape of the sliding contact portion with the inner surface of the barrel was a curved surface with an arc- shape with a chord length of 0.31 mm. All these syringes subjected to the tests had an integrity at room temperature. Further, the cap used in the integrity test was common to all syringes, and a combination cap (made of polyethylene, manufactured by B. Braun Melsungen AG, product number: 4495209) was used. The cap had a shape including a substrate and a mantle tube fitted from a circumferential edge of the substrate to an outer circumferential surface of the spout of the barrel.

### 7-1-2 Test operation: Slidability test

[1] Using a tensile tester (manufactured by A&D Company, Limited TENSILON, RTG-1225), the maximum load (N) at a moving distance of 40 mm at a moving speed of 100 mm/minute was measured. The measurement was performed once with respect to the same syringe.
[2] The slidability of the syringe was determined to be good (Good) when the maximum load was 8 N or less and poor (Poor) when it was greater than 8 N.

### 7-1-3 Test operation: Integrity test

[1] Isopentane (freezing point: about -160°C) was filled into a PE bag.
[2] The gasket was pulled to the display volume of the syringe, the cap was attached without anything stored in the barrel, the syringe was immersed in the isopentane in the PE bag, and the PE bag was closed.
[3] The PE bag was put into a deep freezer (-150°C) and preserved for 3 or more days.
[4] The PE bag was taken out from the deep freezer and immersed in water at room temperature for 5 minutes or more.
[5] The syringe was taken out from the PE bag and lightly washed with water, and then the water was wiped off.
[6] The presence or absence of the intrusion of the isopentane into the barrel of the syringe was visually observed. For each syringe, when the intrusion of the isopentane was confirmed, it was determined to have no integrity (Poor), and when the intrusion of the isopentane was not confirmed, it was determined to have integrity (Good).

Table 10 shows the test results. In the combination of the barrel made of PFA material and the gasket made of HDPE, a syringe with good slidability in which the integrity can be maintained not only at room temperature, but also during any of cooling from room temperature to a very low temperature such as -150°C, preservation at the temperature, and heating from the temperature to room temperature could be obtained.

**Table 10: Evaluation of slidability and evaluation of integrity during cooling and heating (2)**

| | Barrel | | Gasket | | | Ratio | | Slidability | | Integrity |
|---|---|---|---|---|---|---|---|---|---|---|
| Syringe sample No. | Material | Inner diameter (A) (mm) | Material | Diameter (B) (mm) | Thickness (C) (mm) | B/A | C/B | Maximum load (N) | Evaluation | Evaluation |
| 11-1-1 | PFA | 9.10 | HDPE | 9.15 | 0.31 | 1.005 | 0.034 | 4.9 | Good | Good |
| 11-1-2 | PFA | 9.10 | HDPE | 9.15 | 0.31 | 1.000 | 0.034 | 50 | Good | Good |
| 11-1-3 | PFA | 9.10 | HDPE | 9.15 | 0.31 | 1.002 | 0.034 | 6.1 | Good | Good |
| 11-1-4 | PFA | 9.10 | HDPE | 9.15 | 0.31 | 1.002 | 0.034 | 7.1 | Good | Good |

| | | | | | | | | | | |
|---|---|---|---|---|---|---|---|---|---|---|
| Outer diameter of the barrel: 10.6 mm for all syringes The terms in the table indicate the following resins. PFA: Resin R in Table 1 HDPE: Manufactured by Flonchemical, Co., Ltd. | | | | | | | | | | |

### 8. Evaluation of integrity during cooling and heating (3)

In order to determine whether or not the syringe of the present invention can withstand a practical use for cryopreservation at -80°C by evaluating the integrity during cooling and heating of the syringe when storing liquid in the syringe, the following tests for integrity were conducted.

### 8-1-1 Test sample

The barrels and gaskets shown in Table 11 were produced by injection molding and subjected to the tests. These were 2.5 mL volume syringes each consisting of a barrel, a gasket composed of one gasket lip, a plunger, and a finger grip. The gasket had a discotic shape with uniform thickness of 0.31 mm, and the shape of the sliding contact portion with the inner surface of the barrel was a curved surface with an arc- shape with a chord length of 0.31 mm. All these syringes subjected to the tests had an integrity at room temperature. Further, the cap was common to all syringes, and a cap produced by injection molding (made of PE) was used. The cap had a shape including a substrate and a mantle tube fitted from a circumferential edge of the substrate to an outer circumferential surface of the spout of the barrel.

### 8-1-2 Test operation: Integrity test

[1] A yellow pigment (Yellow No.4) was dissolved in 99.5% ethanol to fabricate a colored ethanol (freezing point: about -120°C). The colored ethanol was filled into a PE bag.
[2] 2.5 mL of water was filled in the syringe, and adjusted so that the head space becomes within 0.1 to 0.15 mL. The cap was attached, the syringe was immersed in the colored ethanol in the PE bag, and the PE bag was closed.
[3] The PE bag was put into a deep freezer (-80°C) and preserved for 3 or more days.
[4] The PE bag was taken out from the deep freezer and heated in a hot water bath at 37°C for 5 minutes.
[5] The syringe was taken out from the PE bag and lightly washed with water, and then the water was wiped off.
[6] The colored ethanol was diluted using water, and an aqueous solution with a concentration of 1 v/v%, 0.75 v/v%, 0.5 v/v%, 0.25 v/v%, 0.1 v/v% was prepared.
[7] The wavelength of the spectrophotometer was set to 428 nm, and the absorbance of each solution prepared in [6] was measured to prepare a standard curve. Water was used as control.
[8] Similarly, the wavelength of the spectrophotometer was set to 428 nm, and the absorbance of the filled water of each syringe used in the test was measured, and the concentration of the colored ethanol was calculated using the standard curve.

Table 12 shows the test results. The filled water of any of the syringes subjected to the tests showed an equal absorbance as water used as control. From these results, it has been revealed that there was no intrusion of colored ethanol in the syringe. Therefore, it was shown that all syringes had maintained integrity not only at room temperature, but also during any of cooling from room temperature to a very low temperature such as -80°C, preservation at the temperature, and heating from the temperature to 37°C.

**Table 11: Evaluation of integrity during cooling and heating of the syringe (3) sample**

| | Barrel | | Gasket | | | Ratio | |
|---|---|---|---|---|---|---|---|
| Syringe sample No. | Material | Inner diameter (A) (mm) | Material | Diameter (B) (mm) | Thickness (C) (mm) | B/A | C/B |
| 12-1-1 | PFA | 9.10 | HDPE | 9.15 | 0.31 | 1.005 | 0.034 |
| 12-1-2 | PFA | 9.10 | HDPE | 9.15 | 0.31 | 1.000 | 0.034 |
| 12-1-3 | PFA | 9.10 | HDPE | 9.15 | 0.31 | 1.002 | 0.034 |
| 12-1-4 | PFA | 9.10 | HDPE | 9.15 | 0.31 | 1.002 | 0.034 |

| | | | | | | | |
|---|---|---|---|---|---|---|---|
| Outer diameter of the barrel: 10.6 mm for all syringes The terms in the table indicate the following resins. PFA: Resin R in Table 1 HDPE: Manufactured by Flonchemical, Co., Ltd. | | | | | | | |

**Table 12: Evaluation of integrity during cooling and heating of the syringe (3) results**

| Sample name | Absorbance (Abs) |
|---|---|
| Water (control) 1 | 0.000 |
| Colored ethanol 1v/v% aqueous solution | 0.067 |
| Colored ethanol 0.75v/v% aqueous solution | 0.051 |
| Colored ethanol 0.5v/v% aqueous solution | 0.034 |
| Colored ethanol 0.25v/v% aqueous solution | 0.018 |
| Colored ethanol 0.1v/v% aqueous solution | 0.006 |
| Filled water of syringe sample No. 12-1-1 | -0.003 |
| Filled water of syringe sample No.12-1-2 | 0.000 |
| Filled water of syringe sample No.12-1-3 | 0.000 |
| Filled water of syringe sample No.12-1-4 | 0.000 |
| Water (control) 2 | 0.000 |

### 9. Cell preservation test

In order to determine whether or not the syringe of the present invention can withstand a practical use for cryopreservation of cells at -80°C, the following tests were conducted.

### 9-1-1 Test sample

The barrels and gaskets shown in Table 13 were produced by injection molding and subjected to the tests. These were 2.5 mL volume syringes each consisting of a barrel, a gasket composed of one gasket lip, a plunger, and a finger grip. The gasket had a discotic shape with uniform thickness of 0.31 mm, and the shape of the sliding contact portion with the inner surface of the barrel was a curved surface with an arc-shape with a chord length of 0.31 mm. All these syringes subjected to the tests had an integrity at room temperature. Further, the cap was common to all syringes, and a cap produced by injection molding (made of PE) was used. The cap had a shape including a substrate and a mantle tube fitted from a circumferential edge of the substrate to an outer circumferential surface of the spout of the barrel.

### 9-1-2 Test operation: Cell culture

Human adipose derived stem cells (hAD-MSC; manufactured by Lonza Walkersville; Lot:19TL200176) were seeded and subcultured according to a conventional method. Specifically, by using a hADSC culture kit (ADSC-BulletKit, ADSC-Apidose-Derived Stem Cells Growth Medium BulletKit, manufactured by Lonza Walkersville), 15 mL of hADSC culture was put into a 75 cm² flask, incubated at 37°C with a 5% CO₂ incubator, and subcultured at 80 to 100% confluence.

### 9-1-3 Test operation: Cell preservation test

[1] Human adipose derived stem cells after subculture was suspended in cell preservative solution (STEM-CELLBANKER^{®}GMP grade) to prepare a cell suspension solution (5 × 10⁵ cells/mL).
[2] 2 mL of the cell suspension solution was filled in the syringe (1 × 10⁶ cells/syringe), and adjusted so that the head space becomes within 0.1 to 0.15 mL. The cap was attached.
[3] Each syringe was packaged one by one with an PE outer bag, closed with the air removed from the outer bag.
[4] The PE outer bag was put into a deep freezer (-80°C) and preserved for 10 or more days.
[5] The PE outer bag was taken out from the deep freezer and heated in a hot water bath at 37°C for 1 to 2 minutes.
[6] The syringe is taken out from the PE outer bag, the cap was detached, and the cell suspension solution was poured from the spout of the barrel by pushing the plunger.
[7] 20 µL of the poured cell suspension solution was mixed with 20 µL of 0.4% Trypan blue (Trypan Blue Solution, 0.4%; manufactured by Life Technologies).
[8] The total number of cells, the number of dead cells, and the total cell concentration of the cell suspension solution were measured with a cell counting chamber under a microscope, and the cell viability, the cell recovery rate, and the living cell recovery rate were calculated as follows, respectively. Cell viability (%): (Total number of cells - number of dead cells)/ total number of cells × 100 Cell recovery rate (%) : (total cell concentration after cryopreservation)/ (total cell concentration before cryopreservation) × 100 Living cell recovery rate (%) : Cell viability (%) × Cell recovery rate (%)/100

Table 14 shows the test results. When cryopreserving cells in any of the syringes subjected to the tests, the cell viability, the cell recovery rate and the living cell recovery rate showed a very high level of approximately 100%. It is thought that the error when measuring the cell number caused the cell recovery rate to be over 100% in some of the tests, and it was determined that a number of cells equal to before cryopreservation could be recovered. Further, by visually observing after the test operation [5] of the above 9-1-3, no abnormality such as leakage of cell suspension solution was observed in any of the syringes. Further, when spouting the cell suspension solution from the syringe in the test operation [6] in the above 9-1-3, when pushing the plunger, the gasket smoothly slid, and it was thought that the usability was good. By observing the spouted cell suspension solution with a microscope, there was no abnormality in the cells, and no foreign matter contamination was observed. Therefore, a syringe that while being possible to suppress absorption of the preserved cells to the syringe and death of cells as much as possible (that is, being possible to recover living cells at an approximately maximum rate), in which the integrity can be maintained not only at room temperature, but also during any of cooling from room temperature to a very low temperature such as -80°C, preservation at the temperature, and heating from the temperature to 37°C, and with good slidability could be obtained. From these results, it was shown that the syringe of the present invention could be particularly preferably used for cryopreservation of not only cells but also biological materials such as nucleic acid or protein which similarly require freezing at a very low temperature. Further, since the slidability at actual use was good, it can be said that when attaching an injection needle to the syringe of the present invention to directly administer the contents from the syringe to the subject of administration such as human, it is possible to administer without imposing strain to the body of the subject of administration, and the spout amount could be readily adjusted. Therefore, it was revealed that the syringe of the present invention can be preferably used particularly for medical use such as regenerative medicine (for example, administer therapeutic cells to human).

**Table 13: Cell preservation test sample**

| | Barrel | | Gasket | | | Ratio | |
|---|---|---|---|---|---|---|---|
| Syringe sample No. | Material | Inner diameter (A) (mm) | Material | Diameter (B) (mm) | Thickness (C) (mm) | B/A | C/B |
| 13-1-1 | PFA | 9.10 | HDPE | 9.15 | 0.31 | 1.005 | 0.034 |
| 13-1-2 | PFA | 9.10 | HDPE | 9.15 | 0.31 | 1.000 | 0.034 |
| 13-1-3 | PFA | 9.10 | HDPE | 9.15 | 0.31 | 1.002 | 0.034 |

| | | | | | | | |
|---|---|---|---|---|---|---|---|
| Outer diameter of the barrel: 10.6 mm for all syringes The terms in the table indicate the following resins. PFA: Resin R in Table 1 HDPE: Manufactured by Flonchemical, Co., Ltd. | | | | | | | |

**Table 14: Cell preservation test results**

| | Before cryopreservation | After cryopreservation | | |
|---|---|---|---|---|
| | | Syringe sample No.13-1-1 | Syringe sample No.13-1-2 | Syringe sample No.13-1-3 |
| Total cell concentration (cell/mL) | 4.97× 10⁵ | 4.97× 10⁵ | 5.07× 10⁵ | 5.05× 10⁵ |
| Cell viability (%) | 98.3 | 97.3 | 98.7 | 98.0 |
| Cell recovery rate (%) | - | 100.0 | 102.0 | 101.7 |
| Living cell recovery rate (%) | - | 97.3 | 100.7 | 99.7 |

| | | | | |
|---|---|---|---|---|
| Values in the table all represent mean values of n=3. | | | | |

### Explanation of Letters or Numerals

- 10:: Barrel
- 12:: Cylindrical body
- 14:: Spout
- 15:: Outer circumferential surface of spout
- 16:: Inner circumferential surface of spout
- 17:: Opening
- 18:: Finger grip
- 20:: Gasket
- 22:: Gasket lip
- 30:: Plunger
- 40:: Cap
- 42:: Substrate
- 43:: Circumferential edge of substrate
- 44:: Mantle tube
- 46:: Projection
- 50:: Contents
- 60:: Outer bag
- 100:: Syringe

## Claims

1. A syringe for cryopreservation, comprising:
a barrel having a spout and an opening;
a gasket slidably accommodated in the barrel;
a plunger connected to the gasket; and
a cap that seals the spout of the barrel,
wherein the opening of the barrel is sealed only with the gasket.

2. The syringe according to claim 1, wherein the plunger is connected to the gasket while having a gap from the barrel.

3. The syringe according to claim 1, wherein the cap comprises a substrate and a mantle tube fitted from a circumferential edge of the substrate to an outer circumferential surface of the spout of the barrel.

4. The syringe according to claim 3, wherein the cap further comprises a projection protruding from an inner center of the substrate and fitted into an inner circumferential surface of the spout of the barrel.

5. The syringe according to claim 4, wherein the mantle tube of the cap protrudes towards the barrel side more than the projection of the cap.

6. The syringe according to claim 1, wherein a maximum load when the gasket is slid in the barrel at a speed of 100 mm/minute is 8 N or less.

7. The syringe according to claim 1, wherein a ratio (B/A) of a diameter (B) of the gasket to an inner diameter (A) of the barrel is 1 ≤ (B/A) < 1.050.

8. The syringe according to claim 1, wherein the gasket comprises two or more gasket lips.

9. The syringe according to claim 1, wherein a ratio (X/Y) of a shrinkage rate (X) at which a resin forming the barrel is cooled from a room temperature to a certain temperature to a shrinkage rate (Y) at which a resin forming the gasket is cooled from the room temperature to the certain temperature is 1 ≤ (X/Y) < 1.3.

10. The syringe according to claim 1, wherein the barrel is formed from a material having an absorbed energy in a tensile impact test under liquid nitrogen cooling conditions of 0.23 J or more.

11. The syringe according to claim 1, wherein the barrel and the gasket are each independently formed from a fluororesin or a polyethylene resin.

12. The syringe according to claim 1, wherein the gasket and the plunger are integrally molded.

13. The syringe according to claim 1, wherein the syringe further comprises a finger grip.

14. The syringe according to claim 13, wherein the finger grip is detachable.

15. The syringe according to claim 13, wherein the outer circumferential surface of the finger grip and the outer circumferential surface of the barrel are partially substantially flush with each other.

16. The syringe according to claim 1, wherein the syringe is further stored in an outer bag.

17. The syringe according to claim 1, wherein the syringe is for storing a cell, a nucleic acid, or a protein.

18. The syringe according to claim 17, wherein the cell is a mammalian cell.

19. The syringe according to claim 1, wherein the syringe is for use in regenerative medicine.

20. The syringe according to claim 1, wherein the syringe is used at -80°C or less for cryopreservation.

21. The syringe according to claim 1, wherein the syringe is used at -150°C or less for cryopreservation.

22. A prefilled syringe for cryopreservation, wherein the prefilled syringe stores a content in the syringe according to any one of claims 1 to 21.

23. The prefilled syringe according to claim 22, wherein the prefilled syringed is used at -80°C or less for cryopreservation.

24. The prefilled syringe according to claim 22, wherein the prefilled syringed is used at -150°C or less for cryopreservation.

25. A method for cryopreserving a subject substance, comprising cryopreserving the subject substance in the syringe according to any one of claims 1 to 21.

26. The method according to claim 25, wherein the cryopreservation is a cryopreservation at -80°C or less.

27. The method according to claim 25, wherein the cryopreservation is a cryopreservation at -150°C or less.
